# FASCICULE DE BREVET EUROPEEN

(11) **EP 1 737 856 B1**
(45) Date de publication et mention de la délivrance du brevet: **17.11.2010**
(21) Numéro de dépôt: 05757149.9
(22) Date de dépôt: 14.04.2005
(51) Int. Cl.: C07D 461/00, A61K 31/437, A61P 25/24, A61P 25/18

(54) **DERIVES DU 14.15-DIHYDRO 20.21-DINOREBURNAMENIN-14-OL, ET LEURS APPLICATIONS**
14,15-DIHYDRO-20,21-DINOREBURNAMENIN-14-OL-DERIVATE UND DEREN ANWENDUNG
DERIVATIVES OF 14.15-DIHYDRO 20.21-DINOREBURNAMENIN-14-OL, AND APPLICATIONS THEREOF

(30) Priorité: 14.04.2004 FR 0403873
(43) Date de publication de la demande: 03.01.2007
(73) Titulaire: Biocortech, 75013 Paris (FR)
(72) Inventeur: CIAPETTI, Paola, F-67120 Altorf (FR); DEYON, Laurence, F-67118 Geispolsheim (FR); WERMUTH, Camille-Georges, F-67100 Strasbourg (FR); PUJOL, Jean-François, F-75007 Paris (FR); WEISSMANN, Dinah, F-75007 Paris (FR)
(74) Mandataire: Warcoin, Jacques
(86) Numéro de dépôt international: PCT/FR2005/000902
(87) Numéro de publication internationale: WO 2005/103047

(56) Documents cités:
- EP-A- 0 317 426
- EP-A- 0 317 427
- FR-A- 2 190 113
- FR-A- 2 285 882
- FR-A- 2 312 245
- FR-A- 2 381 048

## Description

L'invention a pour objet de nouveaux dérivés du 14,15-dihydro 20,21-dinoréburnaménin-14-ol, et leurs applications en tant que médicament chez l'Homme.

La dépression est l'un des troubles psychologiques les plus fréquents. En France, le taux de dépressifs est de 14,9 % dont près d'un tiers n'est pas pris en charge médicalement. Une femme sur cinq serait touchée. La prévalence de la dépression déclarée a été multipliée par 6 depuis 1970. Entre 1992 et 1997, le taux de dépressifs a particulièrement augmenté chez les jeunes de 20 à 29 ans (+ 65 %). Il est donc particulièrement indispensable de trouver des traitements mieux adaptés aux dépressions d'autant que certains patients peuvent ne pas répondre aux antidépresseurs classiques.

Des dérivés de la 20,21-dinoréburnaménine, dont le 14,15-dihydro 20,21-dinorébumaménin-14-ol, sont déjà connus pour leurs propriétés vasodilatatrices, notamment cérébrales et pour leur activité dans la régulation de la tyrosine hydroxylase dans le locus coeruleus (Bourde et al., Neurochem. Int., 23 (6), 567-574, 1993). Ils sont utilisés dans les vasculopathies cérébrales et tous les syndromes provoqués par une altération de la circulation cérébrale. Ces dérivés ainsi que leur première application thérapeutique connue ont été décrits dans la demande de brevet FR 2 381 048, publiée le 15 septembre 1978. Cette demande de brevet a fait l'objet d'une demande de certificat d'addition FR 2 433 528 publiée le 14 mars 1980.

Plus particulièrement, la demande FR 2 381 048 décrit les dérivés de la 20,21-dinoréburnaménine et leur procédé de préparation. Sont également décrites les propriétés pharmacologiques de ces composés: ces composés sont des oxygénateurs et vasorégulateurs cérébraux de grande valeur, qui entraînent en particulier une augmentation du flux cérébral au niveau de la microcirculation cérébrale. D'autre part, la demande FR 2 433 528 décrit le procédé de préparation d'un isomère particulier dérivé de la 20,21-dinoréburnaménine, et l'isomère obtenu par ce procédé.

La demande WO 89/04830, publiée le 1er juin 1989, décrit de nouveaux dérivés substitués de la 20,21-dinoréburnaménine, leur procédé de préparation et leur application comme médicament notamment comme antidépresseur.

La dépression est un état psychique pathologique associant une modification pénible de l'humeur et un ralentissement de l'activité intellectuelle et motrice. C'est un état morbide, plus ou moins durable, caractérisé par la tristesse et une diminution du tonus de l'énergie. Une dépression bipolaire se caractérise par des phases alternées : abattement marqué et inertie dans la première, euphorie et hyperactivité dans la seconde.

Les principaux symptômes permettant de diagnostiquer une dépression chez une personne sont une humeur dépressive, une diminution marquée de l'intérêt ou du plaisir, des troubles de l'alimentation, des troubles du sommeil, une agitation ou un ralentissement psychomoteur, une fatigue ou une perte d'énergie, une autodévalorisation ou un sentiment de culpabilité excessive, une diminution de l'aptitude à penser ou à se concentrer ou une indécision, des pensées morbides (dans 60 % des cas), des pensées suicidaires (dans 15 % des cas).

Parmi les facteurs causals de la dépression, on peut citer :

### 1 / Le facteur héréditaire

Les personnes dont les parents proches souffrent ou ont souffert d'une dépression sont plus susceptibles d'en être atteintes. Elles ont 15 % de risque de développer une dépression alors que les personnes dont les parents proches ne sont pas dépressifs ont seulement 2 à 3 % de risque d'en développer une.

### 2/ Le facteur biochimique

Les recherches actuelles sur la dépression portent sur les neurotransmetteurs. On a ainsi pu remarquer qu'une déficience ou un déséquilibre de la sérotonine entraînait une perte de sommeil ainsi qu'une diminution de l'appétit. Mais aussi qu'une baisse de la noradrénaline influe sur la perte d'énergie, le manque de plaisir.

### 3/ Les facteurs liés à l'environnement

Les enfants ayant vécu la perte d'un être cher comme leurs parents, sont plus sujets à développer des dépressions plus tard dans leur vie. Des difficultés dans les relations, des problèmes de communication ainsi que des conflits familiaux, professionnels ou autres, peuvent aussi contribuer à la solitude, à l'aliénation et aboutir à la dépression. Les difficultés financières et autres tensions peuvent également avoir un impact important.

Il ne faut pas négliger les facteurs saisonniers : le taux de dépression est plus élevé au cours des mois où l'ensoleillement est le plus faible. La dépression saisonnière ne se manifeste que pendant la période de l'année où les jours sont courts, ainsi elle se ressent en hiver et disparaît dès le printemps. Elle se manifeste par de la fatigue, un abattement, une absence de tonus et une perte d'intérêt, des troubles de la concentration et de la libido, des fringales pour ce qui est sucré, un besoin de sommeil accru ou encore une prise de poids pendant l'hiver. C'est d'ailleurs pour cela qu'on l'appelle parfois dépression hivernale. 2 % des adultes en Europe centrale sont touchés par la dépression saisonnière et les femmes sont 4 fois plus souvent concernées que les hommes.

La dépression s'accompagne souvent d'autres troubles psychologiques ou accompagne elle-même un autre trouble psychologique. Les crises aiguës d'angoisse et les troubles obsessionnels sont les plus fréquents.

La schizophrénie est une psychose chronique caractérisée par une dissociation psychique ou par une discordance, qui perturbe le cours de la pensée (elle devient hermétique et chaotique), altère le comportement (qui devient étrange, autistique) et bouleverse l'affectivité (archaïque et paradoxale), associée à un délire abstrait et symbolique qui élabore des thèmes d'influence alimentés par des hallucinations auditives et cénesthésiques et vécus dans une atmosphère de dépersonnalisation.

La maladie maniaco-dépressive (dépression bipolaire) et la schizophrénie, qui sont deux maladies mentales, posséderaient la même origine génétique : l'expression de divers gènes intervenant dans certaines cellules du cerveau ainsi que dans la mise en place de la myéline, qui assure la propagation des signaux électriques, serait diminuée. Bien que ces deux maladies présentent un parcours clinique différent, elles partagent certains symptômes, et des médicaments similaires sont très souvent utilisés pour les traiter.

L'art antérieur connaît deux grands types de traitements pour la dépression et la schizophrénie : le traitement médicamenteux et les psychothérapies. Le traitement médicamenteux, qui consiste en une utilisation d'antidépresseurs, est indiqués dans toutes les formes de dépressions. Les antidépresseurs agissent sur l'équilibre des neurotransmetteurs. Les psychothérapies viennent en aide aux malades mais ne peuvent agir comme seul traitement. Il existe d'autres formes de traitements comme les thérapies comportementales et cognitives (concerne surtout les dépressions névrotiques), la sismothérapie ou électrochoc (utilisée en dernier recours).

L'évolution de la dépression est très variable et fonction de nombreux paramètres : étiologie, personnalité du sujet, ....

En dehors de tout traitement, il arrive couramment que la dépression dure 6 mois voire plus avec dans certains cas, comme terminaison extrême, le suicide. Jusqu'à 15 % des sujets présentant un trouble dépressif majeur sévère se suicident. Après traitement, on observe une rechute dans environ 60% des cas.

La dépression peut être diagnostiquée par les critères du DSM IV (Diagnostic and Statistical Manual of Mental Disorders, 4th edition, American Psychiatric Association Publisher ; Washington DC)) ; le DSM IV est un référentiel diagnostique et statistique des troubles mentaux élaboré par l'American Psychiatry Association. Selon les critères du DSM IV, la dépression majeure qui est la forme sévère et la plus commune de la dépression et pour laquelle seulement 10 à 25 % des patients recherchent un traitement, est caractérisée par un ou plusieurs épisodes de changement d'humeur ou de perte d'intérêt d'au moins deux semaines accompagnée d'au moins quatre symptômes additionnels de dépression ; ces derniers symptômes peuvent être par exemple un changement de l'appétit, du poids, du sommeil, ou de l'activité psychomotrice ; une diminution de l'énergie, un sentiment de dévalorisation ou de culpabilité, une difficulté à réfléchir, à se concentrer, à prendre des décisions, ou bien des pensées récurrentes de mort, ou l'idéation, des projets, des tentatives de suicide.

Parmi les dépression majeures, on peut citer les dépressions résistant au traitement par antidépresseurs classiques (dénommées TRD pour « Treatment Resistant Depression ») et également les troubles dépressifs récurrents majeurs (dénommés également MRDD pour « Major Recurrent Depressive Disorders »), troubles qui sont associés à des épisodes hypomaniaques:

Les antidépresseurs classiques actuellement et couramment commercialisés appartiennent aux classes principales suivantes : les antidépresseurs tricycliques (TCA), les inhibiteurs de monoamine oxidase (MAO) (MAOIs), les inhibiteurs sélectifs de recapture de la sérotonine (SSRIs), les inhibiteurs de recapture de la noradrénaline et de la sérotonine (SNDRIs), les antidépresseurs sélectifs de la sérotonine et de la noradrénaline (NASSAs) et les modulateurs du récepteur à la sérotonine.

Il reste à pouvoir disposer de composés capables de traiter les dépressions, les troubles d'une dépression majeure chez un patient, qui peut être résistant au traitement par les antidépresseurs classiques décrits précédemment. Ceci est justement l'objet de l'invention décrite et revendiquée ci-après.

Il a été découvert de nouveaux dérivés du 14,15-dihydro 20,21-dinoréburnaménin14-ol qui permettent de traiter des patients atteints de dépression.

Sous un premier aspect, l'invention a donc pour objet des composés de formule (I) 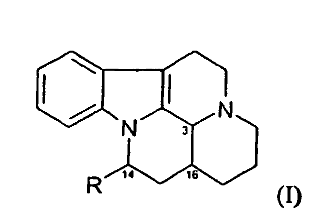 dans laquelle R représente un radical -AR', dans lequel A représente un hétéroatome choisi parmi l'azote ou l'oxygène et R' représente
- un groupement choisi dans le groupe constitué par les radicaux alkyles en C₁-C₆, alcényles en C₁-C₆, alcynyles en C₁-C₆, arylalkyles tels que le benzyle, alcooxyarylalkyles, hétéroarylakyles et hétérocycloalkyles, linéaires ou ramifiés ;
- les esters de formule -R₁-CO-O-R₂, dans laquelle R₁ représente un radical choisi dans le groupe constitué par les radicaux alkyles en C₁-C₆, alcényles en C₂-C₆ ou alcynyles en C₂-C₆, linéaires ou ramifiés, et R₂ représente un radical choisi
- dans le groupe constitué par l'hydrogène, les radicaux alkyles en C₁-C₆, alcényles en C₂-C₆, alcynyles en C₂-C₆ ou cycloalkyles en C₃-C₁₂, avantageusement en C₃-C₆ ou en C₆-C₁₂, linéaires ou ramifiés ;
- les amides de formule 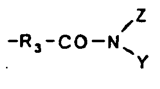 dans laquelle R₃ représente un radical choisi dans le groupe constitué par les radicaux alkyles en C₁-C₆, alcénylènes en C₂-C₆ ou alcynylènes en C₂-C₆, linéaires ou ramifiés, et Y représente un radical choisi dans le groupe constitué par l'hydrogène, les radicaux alkyles en C₁-C₆, alcényles en C₂-C₆, alcynyles en C₂-C₆, aryles, arylaklyles, hétéroarylalkyles ou hétérocycloalkyles, linéaires ou ramifiés, et Z représente un radical choisi dans le groupe constitué par l'hydrogène, les radicaux alkyles en C₁-C₆, alcényles en C₂-C₆, alcynyles en C₂-C₆, aryles, arylaklyles, hétéroarylalkyles ou hétérocycloalkyles, linéaires ou ramifiés, Y et Z pouvant former ensemble un radical cycloalkyle en C₃-C₆ ou un radical hétérocyclique en C₂-C₆, éventuellement substitué par un ou plusieurs radicaux alkyles en C₁-C₆, aryles, hétéroaryles ou halogènes ;
- un radical aminoalkyle choisi dans le groupe constitué par les radicaux alkyles en C₁-C₆, alcényles en C₂-C₆ ou alcynyles en C₂-C₆, linéaires ou ramifiés, substitués par au moins une amine de formule 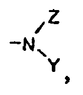 dans laquelle Y et Z sont tels que définis précédemment ;
ou l'un de ses sels pharmaceutiquement acceptables, y compris ses isomères, ses énantiomères, ses diastéréoisomères et leurs mélanges.

Par sels d'addition pharmaceutiquement acceptables, on peut citer ici comme exemple les sels d'addition avec les acides minéraux ou organiques, notamment les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, ascorbique, les acides alcoylmonosulfoniques, tels que l'acide méthanesulfonique, l'acide éthane sulfonique, l'acide propane sulfonique, les acides alcoyldisulfoniques tels que l'acide méthanedisulfonique, l'acide α,β-éthanedisulfonique, et les acides arylmonosulfoniques, tels que l'acide benzènesulfonique et les acides aryldisulfoniques, ces sels n'étant mentionnés qu'à titre illustratif mais sans constituer une limitation.

Le terme « alkyle » désigne un radical hydrocarboné, linéaire ou ramifié, ayant de préférence 1 à 6 atomes de carbones, tel que notamment méthyl, éthyl, propyle, isopropyle, butyle, isobyutyle, tert-butyle, pentyle, n-hexyle.

Les radicaux « aryles » sont des groupes hydrodrocarbonés aromatiques mono- ou bicycliques, généralement à 5 ou 6 chaînons, ayant de 5 à 10 atomes de carbone. Comme exemple de groupe aryle, on peut notamment citer le radical phényle ou naphtyle. Les radicaux « hétéroaryles » sont des groupes hydrodrocarbonés aromatiques présentant sur le ou les cycle(s) au moins un hétéroatome, tel que l'azote, le soufre ou l'oxygène.

Le terme « hétérocycle » désigne des radicaux hydrodrocarbonés aromatiques mono- ou bicycliques présentant sur le ou les cycle(s) au moins un hétéroatome, tel que l'azote, le soufre ou l'oxygène. Les cycles peuvent présenter au moins une insaturation. Comme exemple de radicaux hétérocycliques, on peut notamment citer le radical pipéridine, pipérazine, pyrrolidine, morpholine, homopipérazine, homopipéridine, thiomorpholine, tétrahydropyridine, thiophène, furane, pyridyne, pyrimidine, pyridazine, pyrazine.

Les radicaux « alcoxy » correspondent aux groupes alkyles définis précedemment liés au reste de la molécule par l'intermédiaire d'une liaison éther.

Par « halogène », on entend un atome de fluor, d'iode, de brome ou de chlore. Par « hétéroatome », on entend un atome choisi parmi l'azote, l'oxygène et le soufre.

Les radicaux « arylalkyles », « hétéroarylalkyles » ou « hétérocycloalkyles » sont des groupes comprenant un reste aryle, respectivement hétéroaryle ou hétérocycle, tel que défini ci-dessus lié au reste de la molécule au moyen d'une chaîne alkyle. Comme exemple de radicaux arylalkyles, on peut notamment citer les radicaux benzyle et phénéthyle.

Selon une variante avantageuse de l'invention, R' représente un groupement de formule R₁-CO-O-R₂, dans laquelle R₁ représente un radical alkyle en C₁-C₆, avantageusement le radical -CH₂-. Selon cette variante notamment, R₂ représente avantageusement un atome d'hydrogène, un radical alkyle en C₁-C₆ ou un radical hétérocyclique en C₃-C₆, éventuellement substitué par un ou plusieurs radicaux alkyles en C₁-C₆.

Selon une autre variante avantageuse de l'invention, R' représente un amide de formule 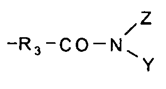 dans laquelle R₃ est tel que défini précédemment et dans laquelle Y et Z forment ensemble un radical hétérocyclique en C₃-C₆, éventuellement substitué par un ou plusieurs radicaux alkyles en C₁-C₆.

Selon une autre variante avantageuse de l'invention, R' représente un radical aminoalkyle choisi dans le groupe constitué par les radicaux alkyles en C₁-C₆ substitués par au moins une amine de formule 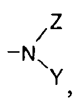 dans laquelle , Y et Z forment ensemble un radical hétérocyclique en C₂-C₆, éventuellement substitué par un ou plusieurs radicaux alkyles en C₁-C₆.

Selon une autre variante avantageuse de l'invention, R' représente un radical hétéroarylalkyle.

Les composés particulièrement avantageux dans le cadre de la présente invention sont les composés de formules suivantes :

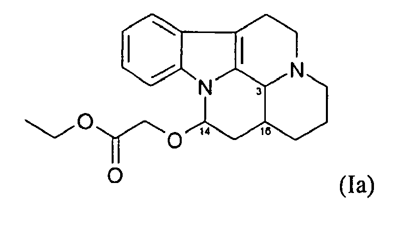

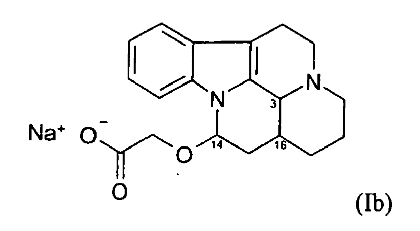

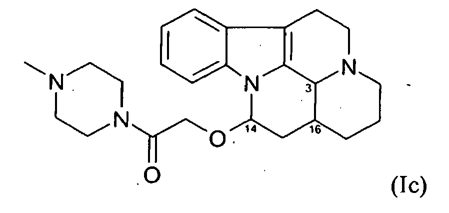

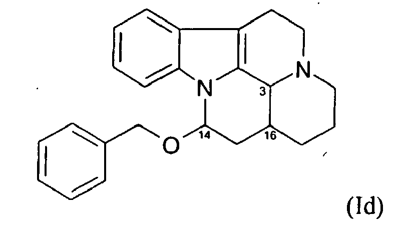

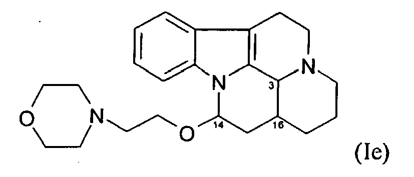

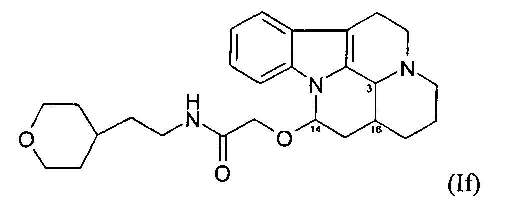

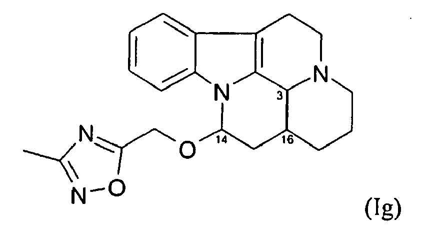

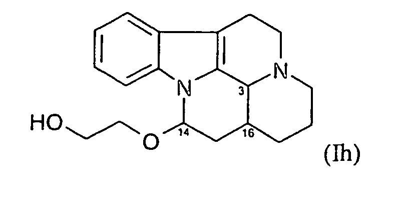

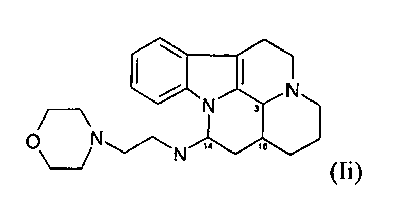

Dans le cadre de l'invention, les composés particulièrement préférés sont les composés (Ia), (Id), (Ie), (If) et (Ig), en particulier le composé (Ie).

Le composé de formule (I) est caractérisé par deux formes énantiomériques 3α et 16α, et le cas échéant est caractérisé pour chacun de ces énantiomères par un couple de diastéréoisomères selon la configuration du carbone 14 : le couple ((3α, 14α) et (3α, 14β)) et le couple ((14α, 16α) et (14β, 16α)).

Dans les composés de formule (I), l'atome d'hydrogène en position 3 et l'atome d'hydrogène en position 16 sont avantageusement en position *trans,* le radical R en position 14 pouvant être quant à lui sous la forme α ou β ( les termes α et β font référence au substituant et non à l'hydrogène, en accord avec la convention de dénomination des dérivés des stéroïdes).

Dans le cadre de la présente invention, la forme 3α correspond aux formules (I) dans lesquelles le carbone en position 3 est de configuration S et le carbone en position 16 est de configuration R. Dans le cadre de la présente invention, la forme 16α correspond aux formules (I) dans lesquelles le carbone en position 3 est de configuration R et le carbone en position 16 est de configuration S.

L'invention a donc pour objet selon un aspect particulier un composé de formule (I) ou l'un de ses sels pharmaceutiquement acceptables dans laquelle le composé de formule (I) ou l'un de ses sels pharmaceutiquement acceptables est sous la forme d'un mélange racémique ou optiquement actif.

Le composé de formule (I) ou l'un de ses sels pharmaceutiquement acceptables est avantageusement choisi parmi les composés de formule (I) suivants :
a) composés sous la forme (3α) dextrogyres et/ou lévogyres ; et
b) composés sous la forme (16α) dextrogyres et/ou lévogyres ,
et dans lequel, le mélange des deux diastéréoisomères lévogyre et dextrogyre présents dans ces composés a) et b) est en proportion équimolaire ou non.

Selon une variante avantageuse de l'invention, le composé de formule (I) ou l'un de ses sels pharmaceutiquement acceptables est avantageusement choisi parmi les composés de formule (I) suivants :
a) composés sous la forme (3α, 14α) dextrogyres et/ou lévogyres ;
b) composés sous la forme (3α, 14β) dextrogyres et/ou lévogyres;
c) composés sous la forme (14α, 16α) dextrogyres et/ou lévogyres; et
d) composés sous la forme (14β, 16α) dextrogyres et/ou lévogyres.

Dans le cadre de la présente invention, on appelle « épimère *trans* » l'épimère dans lequel les atomes d'hydrogènes portés par les carbones 3 et 16 sont toujours en position *trans* et le substituant R est en position *trans* par rapport à l'atome d'hydrogène porté par le carbone 16. Dans le cadre de la présente invention, on appelle « épimère *cis* » l'épimère dans lequel les atomes d'hydrogènes portés par les carbones 3 et 16 sont toujours en position *trans* et le substituant R est en position *cis* par rapport à l'atome d'hydrogène porté par le carbone 16.

Selon une variante avantageuse de l'invention, les composés de formule (I) ou l'un de leurs sels pharmaceutiquement acceptables représentent l'épimère obtenu majoritairement lors de la synthèse, qui peut être l'épimère *trans* ou l'épimère *cis.*

Dans une variante préférée de l'invention, le composé correspond à l'épimère (le1) constitué du couple d'énantiomères (3α, 14β) et (16α, 14α) de formules suivantes :

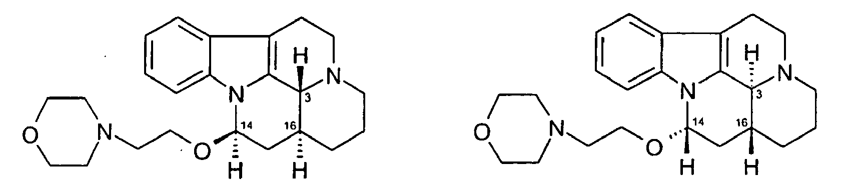

On remarquera que dans cet épimère (Ie1) le substituant porté par l'atome de carbone 14, en l'occurrence l'éthylmorpholine, est toujours en position *trans* par rapport à l'hydrogène porté par l'atome de carbone 16, les atomes d'hydrogènes portés par les carbones 3 et 16 étant toujours *trans.* Plus particulièrement, le composé préféré est l'énantiomère obtenu en second (Ie1b) (second composé élué) en sortie de colonne lorsque ledit épimère (Ie1) (couple (3α, 14β) et (16α, 14α)) est soumis à une chromatographie HPLC (High Performance Liquide Chromatography = Chromatographie Liquide Haute Performance) sur une colonne dont la phase stationnaire est constituée de particules de gel de silice (taille des particules : 5µm) sur lesquelles est greffée de la cellulose tris(2,5-diméthylphénylcarbamate), la phase mobile utilisée étant de l'acétonitrile.

On décrit ici également le composé dans lequel le radical R du composé (I) représente un atome d'hydrogène avec une double liaison entre les carbones 14 et 15, qui répond à la formule (Ij) suivante : 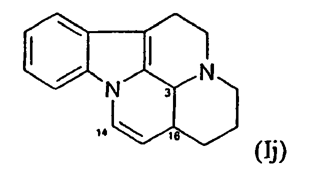 pour son utilisation comme médicament. Le composé (Ij) est notamment obtenu par déshydratation des composés de formule (I), dans lesquels le radical R représente le radical -AR' tel que défini précédemment.

En particulier, on décrit ici le composé (Ij1), qui comprend les deux énantiomères suivants :

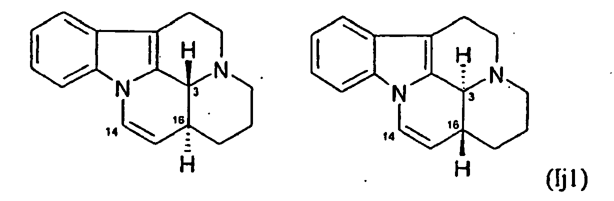

Sous un second aspect, l'invention a pour objet un composé de formule (I) selon l'invention pour son utilisation comme médicament, ainsi qu'une composition pharmaceutique comprenant un composé selon l'invention et un excipient pharmaceutiquement acceptable.

En particulier, la présente invention a pour objet un l'utilisation d'un composé de formule (I) selon l'invention ou d'une composition selon l'invention, pour la préparation d'une composition pharmaceutique destinée au traitement et/ou la prévention de la dépression.

La présente invention a notamment pour objet l'utilisation d'un composé ou d'une composition selon l'invention, pour la préparation d'une composition pharmaceutique destinée au traitement et/ou la prévention des dépressions majeures (MDD) (Réf. 14, 15, 16).

Les composés selon l'invention sont des agents antidépresseurs plus efficaces et ayant une action plus rapide que les antidépresseurs classiques.

Sous un autre aspect, il est décrit ici l'utilisation d'un composé ou d'une composition selon l'invention, le radical R du composé (I) pouvant également représenter un atome d'hydrogène avec une double liaison entre les carbones 14 et 15, destinée au traitement ou à la prévention de patients atteints de dépression et résistant partiellement ou totalement aux traitements par les antidépresseurs classiques (patients atteints de TRD), comme les antidépresseurs appartenant à la classe des antidépresseurs tricycliques (TCA), les inhibiteurs de monoamine oxidase (MAOIs), les inhibiteurs sélectifs de recapture de la sérotonine. (SSRIs) les inhibiteurs de recapture de la noradrénaline et de la sérotonine (SNDRIs), les antidépresseurs sélectifs de la sérotonine et de la noradrénaline (NASSAs) ou les modulateurs du récepteur à la sérotonine.

Sous un autre aspect, il est décrit ici l'utilisation d'un composé ou d'une composition selon l'invention, le radical R du composé (I) pouvant également représenter un atome d'hydrogène avec une double liaison entre les carbones 14 et 15, destinée à rendre sensibles aux traitements antidépresseurs classiques des patients atteints de dépression, notamment de dépression majeure ou sévère, résistant à ces traitements.

Sous un autre aspect particulier, l'invention a pour objet l'utilisation d'un compose ou d'une composition selon l'invention destinée au traitement et/ou à la prévention de la dépression majeure de type bipolaire selon la nomenclature du DSM IV. On peut citer ici notamment un trouble dépressif récurrent majeur (MRDD).

Sous encore un autre aspect, il est décrit ici l'utilisation d'un composé ou d'une composition selon l'invention, le radical R du composé (I) pouvant également représenter un atome d'hydrogène avec une double liaison entre les carbones 14 et 15, destinée au traitement et/ou à la prévention de la dépression dont la sévérité lorsqu'elle est évaluée par l'échelle HAMD (« Hamilton Depression Scale ») a un score supérieur à 26 ou par l'échelle MADRS (Montgomery and Asberg Depression Rating Scale) a un score supérieur à 35.

Sous un autre aspect particulier, l'insertion a pour objet l'utilisation d'un composé ou d'une composition selon l'invention, le radical destinée au traitement et/ou à la prévention de la schizophrénie.

Ainsi, les composés selon l'invention peuvent être utilisés dans le traitement et/ou la prévention de la dépression bipolaire et/ou de la schizophrénie, notamment pour la normalisation des symptomes négatifs des dépressions bipolaires (Réf.17) et des schizophrénies (Réf.18).

Etant donné que les composés selon l'invention, lors de tests sur souris Balb/c, ont été montré capables de : 1) restaurer le phénotype noradrénergique dans une population significative du locus caeruleus ; 2) restaurer l'innervation noradrénergique dans le cortex préfrontal ; 3) restaurer le phénotype hypocretine dans une sous population de neurones de l'hypothalamus ; et 4) inverser l'incapacité de ces souris de race consanguine à présenter un rebond du sommeil REM après privation de sommeil ; l'invention concerne sous un nouvel aspect également l'utilisation desdits composés, pour la fabrication d'un médicament ou pour la préparation d'une composition pharmaceutique destiné à la prévention et/ou au traitement des désordres du cycle veille-sommeil. Lesdits désordres du cycle veille-sommeil sont notamment choisis dans le groupe constitué par la narcolepsie, l'hypersomnie et un état chronique d'hypoéveil.

Sous un nouvel aspect la présente invention a pour objet l'utilisation d'un composé de formule (I) selon l'invention ou d'une composition selon l'invention, le pour la préparation d'une composition pharmaceutique destinée au traitement et/ou la prévention pour le traitement des troubles symptomatiques frontaux dans leur composantes cognitives (Mild Cognitive Impairment (Ref. 18), tels que les états pré-démentiels et démentiels liés aux maladies d'Alzheimer ou de Parkinson (Réf. 12)) ou comportementales. On peut citer ici hypoéveils, troubles de l'attention, troubles caractériels (type ADHD, « ADHD » pour «Attention Deficit-Hyperactivity » Réf.21). Ainsi, sous ce nouvel aspect, la présente invention a pour objet cette utilisation dans laquelle les troubles symptomatiques frontaux dans leur composantes cognitives sont choisis parmi les états pré-démentiels et démentiels liés aux maladies d'Alzheimer et de Parkinson, ou aux maladies comportementales choisies parmi les troubles de l'attention et les troubles caractériels (type ADHD).

Sous un autre aspect particulier, l'invention a pour objet l'utilisation d'un composé de formule (I) selon l'invention ou d'une composition selon l'invention, pour la préparation d'une composition pharmaceutique destinée au traitement et/ou la prévention des troubles de la mémoire, notamment liés au vieillissement ou liés aux maladies d'Alzheimer ou de Parkinson.

De manière plus particulière, l'invention a donc pour objet l'utilisation d'un composé de formule (I) ou l'un de ses sels pharmaceutiquement acceptables selon la présente invention, pour son utilisation comme médicament administrable par voie orale, par voie intraveineuse, par voie intrapéritonéale ou par voie intramusculaire. On peut citer toute autre voie permettant d'obtenir un effet antidépresseur, ou de rendre sensibles aux traitements antidépresseurs classiques les patients atteints de dépression majeure qui étaient résistants à ces traitements, ou encore pour obtenir la prévention ou le traitement désiré dans les utilisations précédentes.

On décrit ici que les substances actives des médicaments ou des compositions pharmaceutiques selon l'invention peuvent être dans n'importe laquelle des formes galéniques orales habituelles comprenant des comprimés, des capsules et des préparations liquides telles que des élixirs et des suspensions contenant diverses substances masquantes de coloration, de saveur et de stabilisation.

Pour réaliser les formes galéniques orales, la substance active peut être mélangée à divers matériaux conventionnels tels que l'amidon, le carbonate de calcium, le lactose, le sucrose et le phosphate dicalcique pour faciliter le processus d'encapsulation. Le stéarate de magnésium, comme additif, fournit une fonction utile de lubrifiant si nécessaire.

Les substances actives des compositions pharmaceutiques peuvent être dissoutes ou mises en suspension dans un liquide stérile pharmaceutiquement acceptable, tel que l'eau stérile, un solvant organique stérile ou un mélange de ces deux liquides. De préférence, un tel liquide est approprié pour l'injection parentérale.

Lorsque la substance active est suffisamment soluble elle peut être dissoute dans une solution saline normale telle qu'un liquide stérile pharmaceutiquement acceptable ; si elle est insuffisamment soluble, elle peut être dissoute dans les solutions aqueuses d'un dissolvant organique approprié, par exemple de glycol de propylène ou de polyéthylène glycol. Le glycol de propylène aqueux contenant de 10 à 75 % en poids du glycol est généralement approprié. Dans d'autres exemples, d'autres compositions peuvent être obtenues en dispersant la substance active en concentré très fin dans la solution carboxyméthylique aqueuse de cellulose d'amidon ou de sodium, ou dans une huile appropriée, par exemple huile d'arachide.

Des compositions pharmaceutiques liquides telles que les solutions ou les suspensions stériles peuvent être utilisées pour l'injection intramusculaire, intrapéritonéale ou sous-cutanée.

La composition pharmaceutique peut être sous la forme de doses unitaires, par exemple comme comprimés ou capsules. Sous une telle forme, la composition est subdivisée dans des doses d'unité contenant des quantités appropriées de la substance active ; les doses unitaires peuvent être les compositions emballées, par exemple des poudres, des fioles ou des ampoules. La quantité de la substance active dans une dose d'unité de composition peut être modifiée ou ajustée de 2 mg ou moins de 50 mg ou plus, selon le besoin particulier et l'activité de la substance active.

Selon la présente invention la dose orale recommandée de composés de formule (I) pour l'homme peut être de 20 à 60 mg/jour. Il est décrit ici que cette dose peut être administrée en deux ou trois doses séparées, de préférence lors d'un repas. La plupart des patients mélancoliques résistants répondent à la dose de 20 mg/jour, mais 40 mg, voire 60 mg peuvent être nécessaires.

L'homme de l'art sait également que les voies d'administration des composés selon cette invention peuvent changer de manière significative. En plus d'autres administrations par voie orale, on peut favoriser des compositions à libération prolongée. D'autres voies d'administration peuvent comprendre, mais ne sont pas limitées, aux injections intraveineuses, intramusculaires et intrapéritonéales, aux implants sous-cutanés, aussi bien que les administrations buccales, sublinguales, transdermiques, topiques, rectales et intranasales.

Selon un mode particulier de réalisation, l'invention a pour objet les composés de formule (I) ou de ses sels pharmaceutiquement acceptables selon l'invention, pour son utilisation comme médicament à des doses journalières de 20 à 60 mg chez l'adulte.

Le spécialiste pourra déterminer le dosage adéquat relatif à chaque patient ; ce dosage pourra varier en fonction de l'âge, du poids et de la réponse au traitement d'un patient donné. Les exemples de dosage ci-dessus sont représentatifs de la moyenne. Il est toutefois possible d'administrer des doses plus ou moins importantes par rapport à cette moyenne.
Procédé de préparation des composés de formule (I) : les composés tels que définis par la formule (I) peuvent être préparés à partir du 14,15 dihydro-20,21-dinoréburnaménin14-ol par les procédés suivants.

### 1) Préparation du 14,15 dihydro-20,21-dinoréburnaménin14-ol :

Le composé 14,15 dihydro-20,21-dinoréburnaménin14-ol est préparé à partir du traitement des composés optiquement actifs de formule (II) ou (II'). 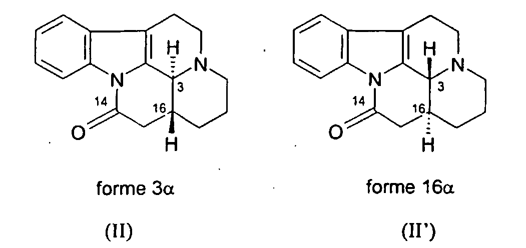 par un agent réducteur ; on obtient les deux couples de diastéréoisomères [(3α, 14α), (3α, 14β)] et [(14α, 16α), (14β, 16α)] du 14,15dihydro-20,21-dinoréburnaménin14-ol, ou leur mélange, et on traite, si désiré, le produit de la réaction par un acide minéral ou organique pour en former le sel.

Les produits de formule (II) et (II') peuvent être préparés par exemple comme indiqué dans la demande de brevet française publiée sous le numéro FR 2 190 113. Le mélange racémique des composés de fonnule (II) peut être séparé par dédoublement.

Par réduction d'un des deux énantiomères de formule (II), on peut obtenir un couple de diastéréoisomères (±) de 14,15 dihydro-20,21-dinoréburnaménin14-ol ou des mélanges en proportions très variables des deux diastéréoisomères. L'expérience décrite dans la demande de brevet français publiée sous le numéro FR 2 623 503 montre que l'on n'obtient pratiquement qu'un seul des deux diastéréoisomères (voir exemple B).

Les composés de formule (II) utilisés peuvent l'être sous forme racémique ou optiquement active. Le ou les composés de réduction du 14,15 dihydro-20,21-dinoréburnaménin14-ol obtenus à partir du produit de formule (II) le sont bien entendu sous la forme stéréochimique correspondante.

Les composés de formule (II) peuvent être utilisés sous forme de l'un de leurs sels d'addition avec les acides minéraux ou organiques. Si tel est le cas, on peut obtenir les produits du 14,15-dihydro-20,21-dinoréburnaménin14-ol sous forme salifiée ou non selon les conditions opératoires choisies.

Les mélanges racémiques ou optiquement actifs des composés du 14,15 dihydro-20,21-dinoréburnaménin14-ol peuvent également être préparés comme indiqué dans la demande de brevet française publiée sous le numéro FR 2 381 048 et dans la demande de certificat d'addition française publiée sous le numéro FR 2 433 528.

Le procédé ci-dessus décrit est réalisé de la manière suivante.

L'agent réducteur utilisé peut être un hydrure, notamment un hydrure mixte, tel que par exemple, l'hydrure mixte de lithium et d'aluminium, le diéthylhydrure de sodium et d'aluminium, l'hydroborure de sodium, l'hydroborure de lithium, l'hydrure de diisobutyl-aluminium. La réaction de réduction est réalisée au sein d'un solvant organique ou d'un mélange de solvants tels que par exemple un éther comme l'éther éthylique, le tétrahydrofuranne, ou un hydrocarbure aromatique comme le toluène, le benzène, le xylène. La réaction de réduction peut être réalisée à une température allant de - 20°C à la température de reflux du milieu réactionnel. Elle peut être réalisée à température ambiante. Dans le cas de l'utilisation comme agent réducteur d'un hydrure métallique, le 14,15 dihydro-20,21-dinoréburnaménin14-ol est libéré du complexe formé intermédiairement avec l'hydrure, selon la pratique courante, par addition d'une solution aqueuse alcaline telle que par exemple une solution d'hydroxyde de sodium.

La réduction du composé (II) *trans* 3α peut conduire au composé (+) (3α, 14α) 14,15-dihydro 20,21-dinorébumaménin 14-ol. La réduction du composé (II') *trans* 16α peut conduire au composé (-) (14β, 16α) 14,15-dihydro 20,21-dinorébumaménin 14-ol.

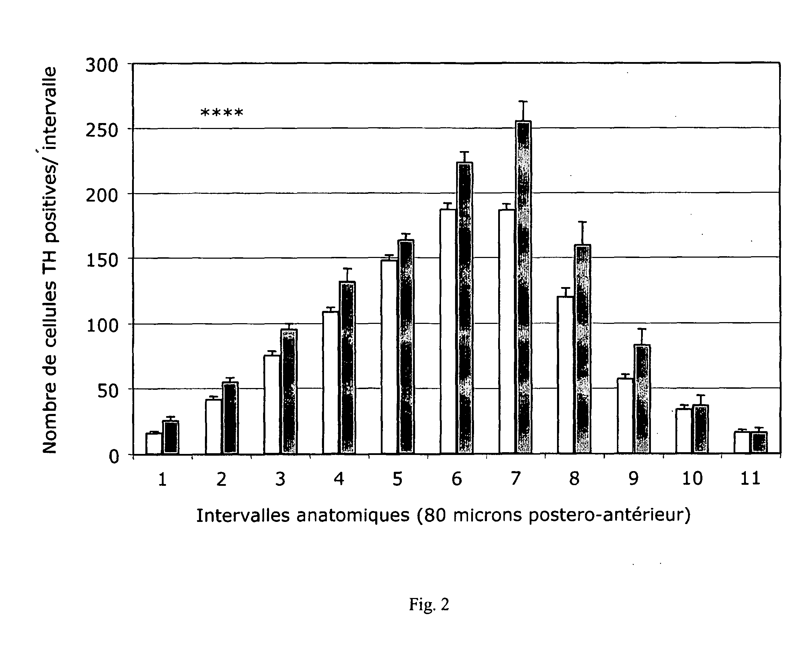

On peut traiter ces composés par un acide; par exemple de l'acide chlorhydrique pour obtenir respectivement les formes (-) (3α, 14β) 14,15-dihydro 20,21-dinorébumaménin 14-ol et (+) (14α, 16α) 14,15-dihydro 20,21-dinoréburnaménin 14-ol majoritaires (cf. schéma ci-après et figure 2).

Schéma représentant la voie générale de synthèse des isomères optiquement actifs des composés du 14,15dihydro-20,21-dinoréburnaménin14-ol à partir des composés de formule (II) (composés de formule (II) décrits dans le document de brevet belge publié sous le N° BE 764 166)

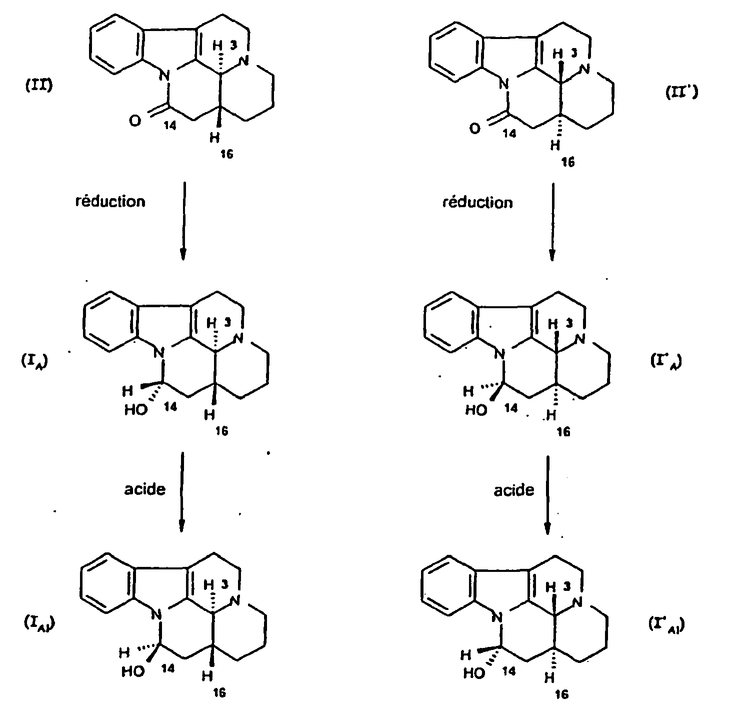

L'isolement de l'un ou de l'autre des diastéréoisomères de leur mélange peut être réalisé par des méthodes usuelles : chromatographie, cristallisation directe, solubilisation différentielle telle que par exemple la solubilisation différentielle dans le toluène à chaud.

### 2) Préparation des composés de formule (I) :

Les composés peuvent être préparés à partir du 14,15 dihydro-20,21-dinorébunaménin14-ol par O-alkylation ou N-alkylation en présence d'une base forte, telle que NaH, ou à partir des composés selon l'invention déjà synthétisés.

Les exemples et les figures qui suivent sont destinés à illustrer l'invention.

### Légendes des figures

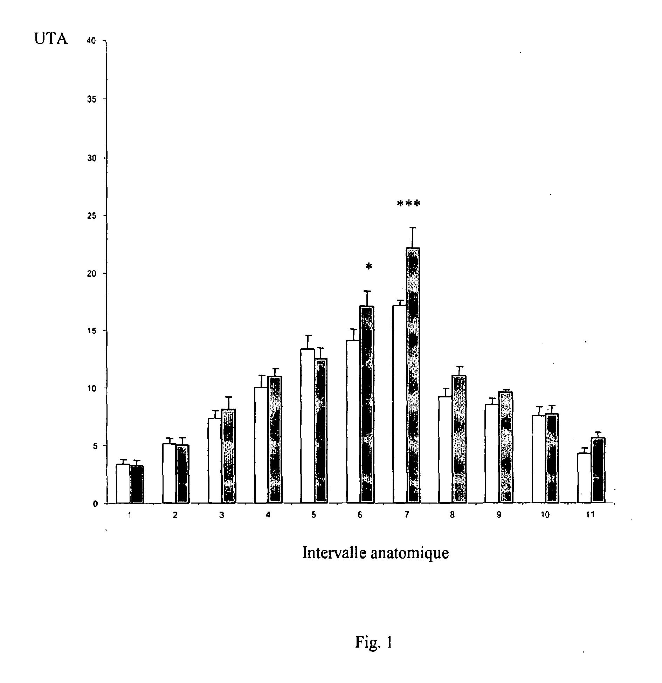

**Figure 1** : la figure 1 représente le contenu en protéine TH (Tyrosine hydroxylase) mesuré après transfert direct de coupes coronales de cerveau congelé sur des filtres de nitrocellulose, exprimé en unité arbitraire (UTA signifie « Unité Tyrosine hydroxylase Arbitraire »), dans chaque intervalle anatomique (80µm), dans un groupe de souris contrôle (barres blanches) et dans un groupe de souris traitées avec le composé de formule (If1) (barres grises).
**Figure 2** : distribution postéro-antérieure du nombre de cellules contenant la TH dans le LC déterminée par immunohistochimie dans un groupe témoin de souris Balb/c (barres blanches) et dans un groupe de souris traitées par la molécule de formule (Ie1) (barres grises).

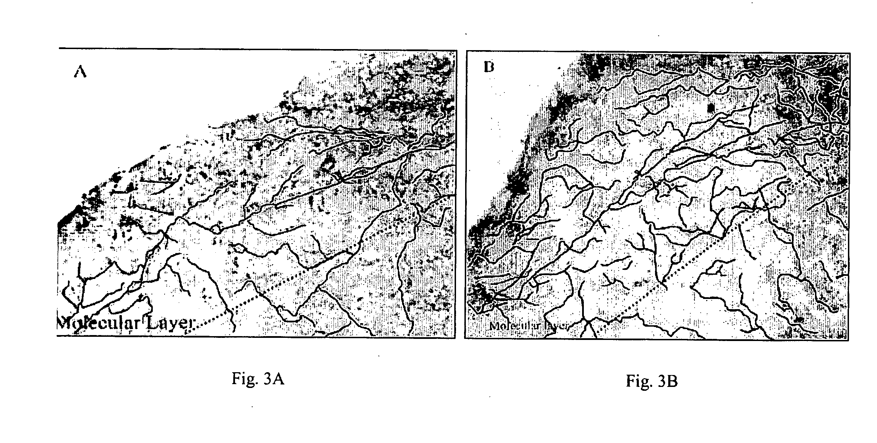

**Figures 3A et 3B** **:** distribution des fibres TH positives sur un échantillon de cortex préfrontal de la souris Balb/c déterminée par immunohistochimie, chez une souris témoin (Figure 3A) et après un traitement séquentiel par la molécule de formule (Ie1) (Figure 3B). Les fibres TH positives ont été surlignées. Sur ces figures, l'indication « Molecular Layer » signifie « Couche moléculaire ».

Dans les exemples 1 à 9 qui suivent :
- l'atome d'hydrogène en position 3 et l'atome d'hydrogène en position 16 sont *trans* (la liaison pouvant être (3β, 16α) ou (3α, 16β))
- en position 14, les termes α et β font référence au substituant et non à l'hydrogène.

De plus, les composés synthétisés, dans les exemples 1 à 9 qui suivent, peuvent être sous la forme soit d'un mélange de deux épimères (c'est à dire quatre diastéréoisomères) soit d'un seul épimère, l'épimère *cis* ou l'épimère *trans* (c'est à dire deux énantiomères). Les deux épimères sont définis en fonction de la position (en avant ou en arrière du plan) du radical -R porté par l'atome de carbone en position 14 et en fonction de sa position par rapport à l'hydrogène porté par le carbone 16.

Pour chaque épimère, on a deux énantiomères en fonction de la position (en avant ou en arrière du plan) des atomes d'hydrogènes en position 3 et 6 (ces atomes d'hydrogène sont toujours *trans*).

| | | |
|---|---|---|
| **Epimère *trans* (2 énantiomères)** | 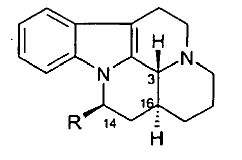 | 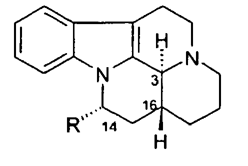 |
| **Epimère *cis* (2 énantiomères)** | 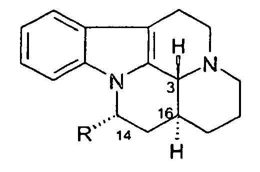 | 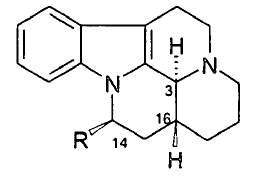 |

Dans les exemples 1 à 9 qui suivent, l'expression « 2 épimères » signifie que le composé (de départ ou synthétisé) est sous la forme d'un mélange des deux épimères (quatre diastéréoisomères) et l'expression « 1 épimère » signifie que le composé (de départ ou synthétisé) est sous la forme d'un seul épimère (deux énantiomères), l'épimère *cis* ou l'épimère *trans.*

### Exemple 1 : Procédé de préparation du composé de formule (Ia) : (±) (16α) 14,15-dihydro 14-éthoxycarbonylméthoxy-20,21-dinoréburnaménine

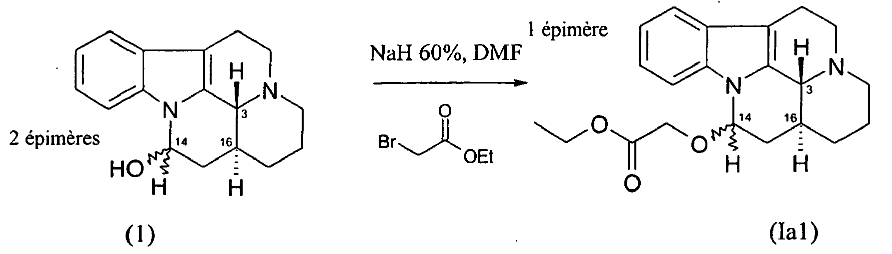

On dissout 50 mg (0,19 mmol) de composé (1) dans 4 ml de diméthylformamide anhydre (DMF) puis on ajoute 19 mg de NaH 60% (1,2 éq). Une fois le dégagement gazeux terminé, on ajoute 25 µl (1,2 éq) de bromoacétate d'éthyle et on agite à température ambiante pendant une nuit. On concentre ensuite le milieu réactionnel, puis on reprend le résidu avec du dichlorométhane (CH₂Cl₂). On lave la phase organique avec de l'eau. On sèche sur sulfate de magnésium (MgSO₄) on filtre et on évapore à sec. On purifie sur colonne de silice éluée avec un mélange CH₂Cl₂/MeOH 99:1 puis CH₂Cl₂/MeOH 98:2. On obtient le composé (Ia) sous forme de 2 épimères : 28 mg d'une poudre blanc-cassé (Ia1) et 3 mg de solide collant jaune (Ia2). Rendement : 46% (42% de Ia1 et 4% de Ia2). Point de fusion = 105°C - 108°C
RMN ¹H CDCl₃ (300 MHz) δ (ppm) : 1,25 (m, 2H, -C*H₂*) 1,27 (t, 3H, -CH₂C*H₃*) ; 1,79 (m, 2H, -C*H₂*) ; 2,33 (m, 4H, 2 *-*C*H₂*) ; 2,72 (m, 3H, *-*C*H₂,* -C*H*) ; 3,12 (m, 3H, -C*H₂,* -CH) ; 4,11 (m, système AB, 2H, -COC*H₂*, J = 15,9 Hz) ; 4,22 (q, 2H, -C*H*₂CH₃) ; 5,77 (m, 1*H*, C*H*OH) ; 7,15 (m, 2H, H *aromatiques*) ; 7,45 (dd, 1H, H *aromatique,* J = 7,22 Hz, J = 1,55 Hz) ; 7,68 (dd, 1H, H *aromatique,* J = 7,03 Hz, J = 1,69 Hz)

### Exemple 2 : Procédé de préparation du composé de formule (Ib1): sodium, (±) (3ß, 16α) 14,15-dihydro 14-carboxyméthoxy 20,21-dinoréburnaménine

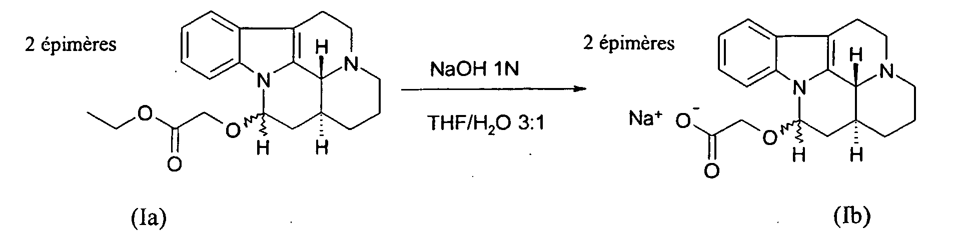

On dissout 182 mg (0,51 mmol) de composé (Ia) dans 8 ml d'un mélange tétrahydrofurane (THF)/H₂O 3:1. On ajoute 485 µl (0,95 éq) d'une solution de soude 1N. On agite une nuit à température ambiante. On concentre le THF, on reprend avec du CH₂Cl₂ et de l'eau. On lave 3 fois la phase aqueuse avec du CH₂Cl₂. On concentre la phase aqueuse et on sèche le solide obtenu. On obtient 160 mg du composé désiré (Ib) sous forme d'un solide jaune. Rendement : 90% Point de fusion = 200°C.
RMN ¹H MeOD (300 MHz) δ (ppm) : 1,31 (m, 1H, -C*H*) ; 1,59 (m, 1H, -C*H*) ; 1,87 (m, 4H, 2 - C*H₂*) ; 2,41 (m, 2H, *-*C*H₂*) ; 2,71 (m, 2H, -C*H₂*) ; 2,93 (m, 3H, -C*H₂, -*C*H*) ; 3,13 (m, 1H, -C*H*) ; 3,92 (m, système AB, 2H, -COC*H₂*, J = 15 Hz) ; 5,70 (m, 1H, C*H*OH) ; 7,07 (m, 2H, H *aromatiques*) ; 7,39 (d, 1H, H *aromatique,* J= 7,1 Hz) ; 7,70 (d, 1H, H *aromatique,* J = 7,9 Hz)

### Exemple 3 : Procédé de préparation du composé de formule (Ic1) : (±) (3β, 16α) 14,15-dihydro 14-[2-(N-méthyl-pipérazin-1-yl)-2-oxo-éthoxyl 20,21-dinoréburnaménine

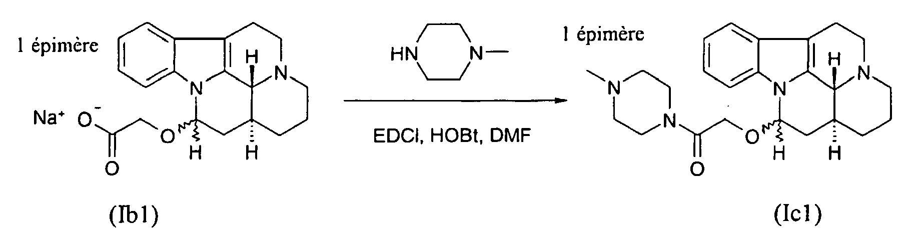

### (Ib1) : épimère majoritaire séparé après synthèse du composé (Ib).

On dissout 80 mg (0,23 mmol) du composé (Ib1) dans 5 ml de DMF. On ajoute 31 µl (1,2 éq) de N-méthylpipérazine, 53 mg (1,2 éq) du chlorhydrate de 1-(3-diméthylaminopropyl-3-éthylcarbodiimide (EDCI.HCl) et 37 mg (1,2 éq) de l'hydrate de 1-hydroxybenzotriazole (HOBt.H₂O). On agite 48h à température ambiante, on concentre le mélange réactionnel. On reprend le résidu avec du CH₂Cl₂, on lave la phase organique avec une solution saturée en NaHCO₃. On sèche sur MgSO₄, on filtre et on concentre. On purifie l'huile jaune obtenue sur colonne de silice éluée avec un mélange CH₂Cl₂/MeOH 95:5 puis CH₂Cl₂/MeOH 90 :10. On obtient 50 mg d'une poudre jaune collante (Ic1). Rendement : 53% Point de fusion = 44°C - 48°C
RMN ¹H CDCl₃ (300 MHz) δ (ppm) : 1,26 (m, 1H, -C*H*) ; 1,60 (m, 1H, -C*H*) ; 1,82 (m, 4H, 2 - C*H₂*) ; 2,34 (m, 6H, 3 -C*H₂*) ; 2,42 (m, 3H, -C*H₂,* -C*H*) ; 2,75 (m, 3H, *-CH₂*, -CH) ; 3,04 (m, 3H, - C*H₂,* -C*H*) ; 3,42 (m, 2H, -C*H₂*) 3,60 (m, 2H, -C*H₂*); 4,19 (m, système AB, 2H, -COC*H₂*, J = 13,5 Hz) ; 5,78 (m, 1H, C*H*OH) ; 7,12 (m, 2H, H *aromatique*) ; 7,44 (d, 1H, H *aromatique*) ; 7,55 (d, 1H, H *aromatique*)

### Exemple 4 : Synthèse du composé (Id) : (±) (3β, 16α) 14,15-dihydro 14-benzyloxy 20,21-dinoréburnaménine

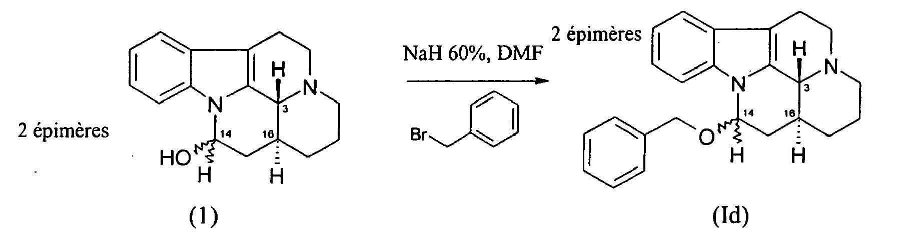

On utilise la même méthode que celle utilisée pour la synthèse du composé (Ia) (exemple 1) en utilisant le bromure de benzyle à la place du bromoacétate d'éthyle. Rendement : 52% (Id) (les deux épimères n'ont pas été séparés). Point de fusion : 112°C - 115°C
RMN ¹H (300 MHz, CDCl₃) δ (ppm) 1,15-1,35 (m, 2H); 1,78-2,02 (m, 4H); 2,18-2,40 (m, 2H); 2,54-2,89 (m, 3H); 2,94-3,21 (m, 3H); 4,48-4,73 (m, 2H); 5,74 (m, 1H); 7,13 (m, 2H); 7,18-7,38 (m, 5H); 7,45 (m, 1H); 7,54 (m, 1H).

### Exemple 5 : Synthèse du composé (Ig) : (±) (3β, 16α) 14,15-dihydro 14-(3-méthyl-[1,2,4]oxadiazol-5-ylméthoxy) 20,21-dinoréburnaménine

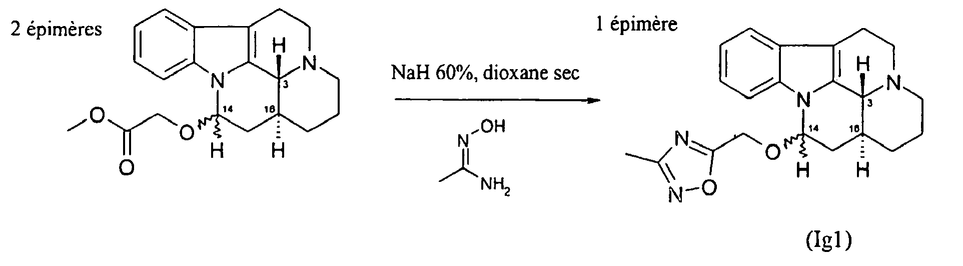

L'ester méthylique de départ a été synthétisé avec la même méthode que celle utilisée pour la synthèse du composé (Ia) en utilisant du bromoacétate de méthyle à la place du bromoacétate d'éthyle.
On dissout 209 mg (2,82 mmol) d'acétamide oxime et 113 mg (2,82 mmol) de NaH 60% dans 10 mL de dioxane anhydre. On ajoute une spatule de tamis moléculaire et on porte le mélange réactionnel à 65°C pendant une heure. On ajoute, à cette température, 320 mg (0,94 mmol) d'ester méthylique dissous dans 10 mL de dioxane anhydre et on porte le mélange réactionnel à 75°C pendant une nuit. On concentre le mélange réactionnel, on reprend le résidu avec une solution non saturée de NaHCO₃, on triture, on filtre. On purifie le solide jaune obtenu sur colonne de silice éluée avec un mélange CH₂Cl₂/MeOH (99:1). Les deux épimères sont séparés. On obtient de 79 mg de l'épimère majoritaire (Ig1) sous forme de poudre blanche. Rendement : 23% pour l'épimère majoritaire, l'épimère minoritaire n'a pas été isolé. Point de fusion : 124°C - 125°C
RMN ¹H (300 MHz, CDCl₃) δ (ppm) 1,21-1,38 (m, 1H); 1,60-1,73 (m, 1H); 1,75-2,03 (m, 4H); 2,29-2,55 (m, 5H); 2,65-2,96 (m, 3H); 2,98-3,23 (m, 3H); 4,68 (d, 1H, J = 14.1 Hz); 4,80 (d, 1H, J = 14.1 Hz); 5,83 (m, 1H); 7,16 (m, 2H); 7,46 (m, 1H); 7,59 (m, 1H).

### Exemple 6 : Synthèse du composé (Ih): (±) (3β, 16α) 14,15-dihydro 14-(2-hydroxy-éthoxy) 20,21-dinoréburnaménine

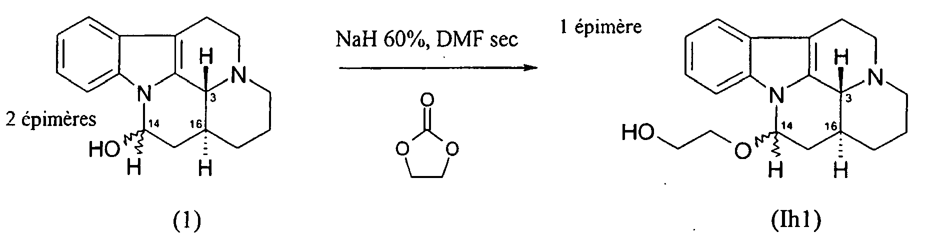

On met en suspension 300 mg (1,12 mmol) du composé (1) dans 10 mL de DMF anhydre puis on ajoute 54 mg (1,2 eq) de NaH 60%. On additionne 492 mg (5,0 eq) de carbonate d'éthylène et on porte le mélange réactionnel une heure à 110°C puis quatre heures à 80°C. On concentre le mélange réactionnel, on reprend le résidu avec du CH₂Cl₂. On lave la phase organique deux fois à l'eau et une fois avec une solution saturée de NaHCO₃. On séche sur MgSO₄, on filtre et on concentre. On purifie l'huile brune obtenue sur une colonne de silice éluée avec un mélange CH₂Cl₂/MeOH (99:1) puis CH₂Cl₂/MeOH (98:2) puis CH₂Cl₂/MeOH (96:4).

On obtient le composé sous forme de 2 épimères : 111 mg (Ih1) de solide beige et 100 mg de solide beige (Ih2). Rendement : 61% (32% de (Ih1) et 29% de l'autre épimère). RMN ¹H (400 MHz, CDCl₃) δ(ppm) 1,22-1,38 (m, 1H); 1,78-2,18 (m, 6H) ; 2,21-2,41 (m, 2H); 2,61 (m, 1H); 2,71-2,85 (m, 2H); 2,97-3,20 (m, 3H); 3,56-3,82 (m, 4H); 5,61 (m, 1H); 7,14 (m, 2H); 7,44 (m, 1H); 7,52 (m, 1H).
Point de fusion : 129°C - 132°C

### Exemple 7 : synthèse du composé (Ie)

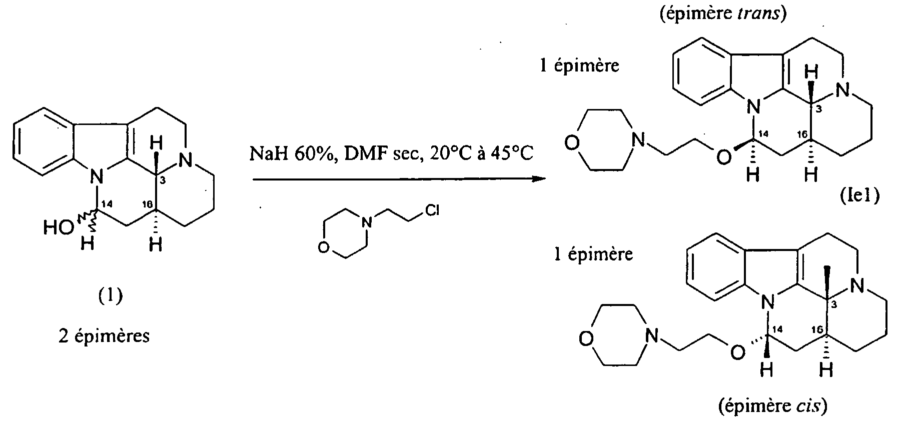

### a) Synthèse 4-(2-chloroéthyl)-morpholine

On dissoud 5,0 g (26,9 mmole) du chlorhydrate de la 4-(2-chloroéthylmorpholine) dans 16 mL d'eau distillée. On ajoute par portions 15 g de carbonate de potassium (K₂CO₃). On extrait la solution aqueuse cinq fois à l'acétate d'éthyle (AcOEt). On séche sur MgSO₄, on filtre et on concentre la phase organique.
On obtient 3,37 g de 4-2(chloroéthylmorpholine) sous forme d'huile jaune clair. Rendement : 84%

### b) Synthèse des composés (Ie) : (±) (3β, 14β, 16α) 14,15-dihydro 14-(2-morpholin-4-yl-éthoxy) 20,21-dinoréburnaménine et (±) (3β, 14α, 16α) 14,15-dihydro 14-(2-morpholin-4-yl-éthoxy) 20,21-dinoréburnaménine

On met en suspension 500 mg (1,86 mmol) du composé (1) dans 20 mL de DMF anhydre. On ajoute 90 mg (1,2 eq) de NaH 60% et on laisse agiter 45 minutes à température ambiante. On ajoute 558 mg (2,0 eq) de 4-(2-chloroéthyl)morpholine dissous dans 10 mL de DMF anhydre puis 140 mg (0,5 eq) de iodure de sodium. On agite 8 heures à 45°C puis on rajoute à cette même température 90 mg (1,2 eq) de NaH 60%, 558 mg (2,0 eq) de 4-(2-chloroéthyl)morpholine dissous dans 2 mL de DMF anhydre et 280 mg (1,0 eq) de iodure de sodium. On poursuit le chauffage pendant 18 heures puis on ajoute à nouveau 90 mg (1,2 eq) de NaH 60%, 558 mg (2,0 eq) de 4-(2-chloroéthyl)morpholine dissous dans 2 mL de DMF anhydre et 280 mg (1,0 eq) de iodure de sodium. On poursuit l'agitation 24h à 45°C. On concentre le mélange réactionnel, on reprend le résidu avec du CH₂Cl₂. On lave trois fois la phase organique à l'eau. On la sèche sur MgSO₄, on filtre et on concentre. On reprend le résidu obtenu avec de l'Et₂O, on filtre l'insoluble, on concentre le filtrat. On purifie l'huile marron obtenue sur colonne de silice éluée avec un mélange CH₂Cl₂/MeOH/NH₂OH (99:0.5:0.5).
Obtention des deux épimères : 340 mg de solide collant jaune (Ie1) et 29 mg (Ie2) de solide jaune. Rendement : 52% (48% de (Ie1) et 4% de (Ie2)).
**(Ie1):**
RMN ¹H (300 MHz, CDCl₃) δ (ppm) 0,86 (m, 1H); 1,18-1,40 (m, 1H); 1,78-1,92 (m, 4H); 2,21-2,41 (m, 2H); 2,48-2,92 (m, 9H); 2,98-3,15 (m, 3H); 3,59 (m, 1H); 3,70-3,92 (m, 5H); 5,62 (m, 1H); 7,08-7,21 (m, 2H); 7,44 (m, 1H); 7,63 (m, 1H).
Point de fusion 116°C - 118°C
**(Ie2)** :
RMN¹H (300 MHz, CDCl₃) δ (ppm) 1,12-1,32 (m, 1H); 1,79-2,82 (m, 16H); 2,95-3,21 (m, 3H); 3,43-3,80 (m, 6H); 5,69 (bs, 1H); 7,11 (m, 2H); 7,34-7,52 (m, 2H).
Point de fusion : 91°C - 93°C

### c) Séparation des deux énantiomères (Ie1a) et (Ie1b) par HPLC chirale préparative

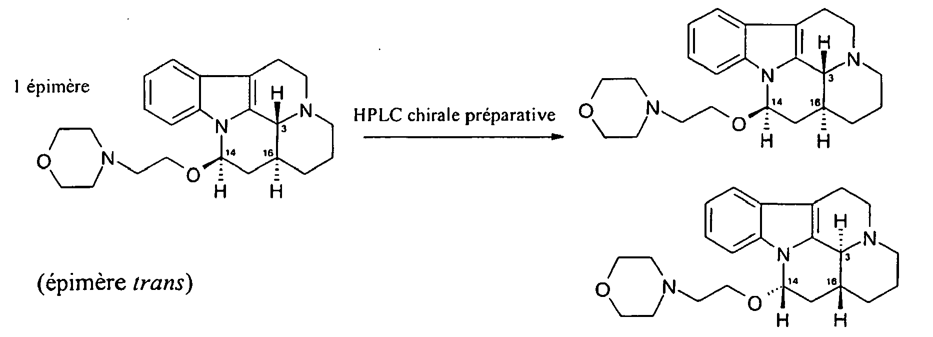

Chaque énantiomèe du composé (Ie1) est séparé par chromatographie préparative en utilisant une colonne CHIRACEL® OD-H sous les conditions suivantes :

### Méthode chromatographie liquide preparative :

Colonne : 250 x 20 mm CHIRACEL® OD-H 5µm
Phase mobile : Acétonitrile
Débit : 20ml/min
Détection: UV 300nm
Température: 25°C

### Méthode chromatographie liquide analytique :

Colonne : 250 x 4,6 mm CHIRACEL® OD-H 5µm
Phase mobile: Acétonitrile
Débit : 1,0ml/min
Détection: UV 230nm
Température: 25°C

### Résultats:

A partir de 582 mg de produit de départ, on obtient les deux énantiomères suivants (Ie1a) et (Ie1b) (tableau 1).

**tableau 1 : caractéristiques des deux énantiomères séparés par HPLC chirale préparative**

| Premier énantiomère élué (**Ie1a**) | | Second énantiomère élué (**Ie1b**) | |
|---|---|---|---|
| Temps de Rétention (min) | 5,87 | Temps de Rétention (min) | 6,80 |
| Quantité (mg) | 265 | Quantité (mg) | 264 |
| Pureté chimique (% surface à 230nm) | 99,3 | Pureté chimique (% surface à 230nm) | 99,8 |
| Excès énantiomérique (%) | >99,5 | Excès énantiomérique (%) | >99,5 |

### Exemple 8 : Synthèse du composé (If1) : (±) (3β, 16α) 14,15-dihydro 14-[(2-morpholin-4-yl-éthylcarbamoyl)-méthoxy] 20,21-dinoréburnaménine

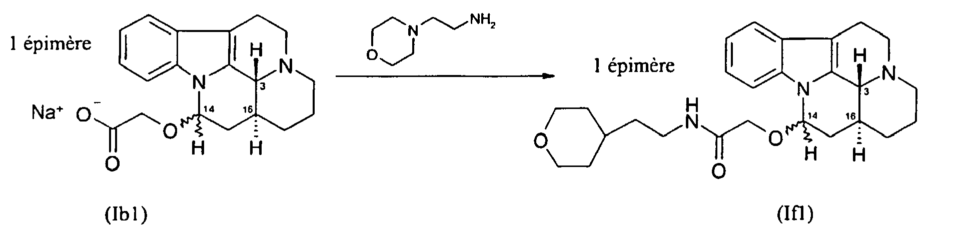

On utilise la même synthèse que pour le composé (Ic) en utilisant la 4-(2-aminoéthylmorpholine) à la place de la N-méthylpiperazine. On obtient 56 mg de poudre jaune (If1). Rendement : 56% Point de fusion : 181 °C - 184°C
RMN ¹H (300 MHz, CDCl₃) δ (ppm) 1,18-1,35 (m, 1H); 1,50-1,71 (m, 1H); 1,75-1,93 (m, 4H); 2,32-2,83 (m, 11H); 2,91-3,17 (m, 3H); 3,38 (m, 2H); 3,55-3,72 (m, 4H); 3,99 (d, 1H, J = 14.57 Hz); 4,12 (d, 1H, J = 14.57 Hz); 5,69 (m, 1H); 7,13 (m, 3H); 7,35-7,52 (m, 2H).

### Exemple 9 : Synthèse du composé (Ii) : (±) (3β, 16α) 14,15-dihydro 14-(2-morpholin-4-yl-éthylamino) 20,21-dinoréburnaménine

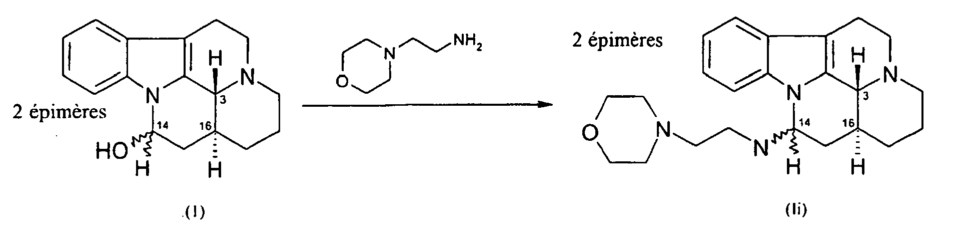

On dissoud 200 mg (0,75 mmol) du composé (1) dans 4 mL de 4-(2-aminoethyl)morpholine et on porte le mélange réactionnel. à 110°C pendant 4 jours. On revient à température ambiante, on reprend le résidu avec du CH₂Cl₂. On lave dix fois la phase organique avec de l'eau. On la sèche sur MgSO₄, on filtre et on concentre. On reprend l'huile brune obtenue avec de l'éther éthylique, on filtre, on concentre le filtrat. On purifie le solide marron obtenu sur une colonne de silice éluée avec un mélange CH₂Cl₂/MeOH (98:2) puis CH₂Cl₂/MeOH (96:4).On obtient 44 mg d'huile jaune collante contenant les deux épimères. Rendement : 15%
RMN ¹H (300 MHz, CDCl₃) δ (ppm) 1,18-1,38 (m, 3H); 1,78-2,81 (m, 17H); 2,93-3,21 (m, 3H); 3,40-3,57 (m, 1H); 3,61-3,78 (m, 3H); 5,18-5,40 (m, 1H); 7,03-7,21 (m, 2H); 7,31-7,64 (m, 2H).

### Exemple 10 : Protocole pharmacologique

Les molécules ont été sélectionnées par rapport à leur capacité à :
1) activer l'expression de la protéine dans le Locus Caeruleus (LC) chez la souris Balb/c ;
2) leur capacité à révéler une population significative de cellules dans laquelle le phénotype de la tyrosine hydroxylase (TH) est restauré par le traitement.

Le contenu en protéine TH est mesuré après transfert direct de coupes coronales de cerveau congelé sur des filtres de nitrocellulose. Un échantillonnage précis de la distribution en protéine, révélé par immunochimie quantitative, a ainsi été effectué chaque 80 µm (définissant ainsi chaque intervalle anatomique). La quantification a été réalisée par utilisation d'une gamme d'homogénats de cerveau contenant une quantité croissante de protéine TH.

Le nombre de cellules immunopositives pour la TH est déterminé à partir de coupes coronales de cerveau fixé par immunohistochimie selon le même échantillonnage anatomique.

Ces analyses sont effectuées avec des animaux traités par un véhicule et avec des souris traitées par une unique injection intrapéritonéale (20mg/kg dans 100 µl) de molécule à tester. Les animaux sont euthanasiés trois jours après l'injection.

Un résultat typique est présenté sur la figure 1 ci-jointe. Il montre clairement que l'augmentation de l'induction du gène de la TH se produit dans un niveau spécifique du noyau. Le contenu en protéine TH a été déterminé 3 jours après injection unique de souris traitées ou de souris contrôle. La quantité en protéine TH a été déterminée dans chaque intervalle anatomique tout le long de l'axe caudo-rostral. Chaque barre représente la valeur moyenne ± sem du groupe contrôle (barres blanches) et du groupe traité (barres grises). Dans la figure, l'astérisque « * » signifie que le résultat est significatif (p<0,05) ; l'astérisque « *** » signifie que le résultat est très significatif (p<0,0005). On a trouvé une augmentation de 13 ± 3% de contenu en TH total dans le LC (p<0001, suivant le test ANOVA II).

### Activité biologique

Toutes les molécules de formules générales (exemple) ont été synthétisées et criblées *ex vivo* sur leur capacité à induire une augmentation de l'expression de la tyrosine hydroxylase (TH) dans le Locus caeruleus (LC) de souris de la souche inbred Balb/c. Ce modèle génétique a été précédemment validé comme un modèle révélateur de la capacité d'une molécule administrée par voie périphérique, à faire apparaître des cellules noradrénergiques « donnantes » (Réf. 1 et 2) Des exemples des résultats de ce criblage sont donnés dans le tableau 2 ci-après.

**Tableau 2**

| **Molécules** | **Quantité de TH mesurée dans le LC** |
|---|---|
| Composé de formule (1) | 120 ± 4 ** |
| Composé de formule (Ia1) | 151 ± 5 ** |
| Composé de formule (Id) | 129 ± 5 ** |
| Composé de formule (Ie1) | 138 ± 4 **** |
| Composé de formule (If1) | 113 ± 4 ** |
| Composé de formule (Igl) | 118 ± 3 ** |
| Composé de formule (Ij1) | 123 ± 2 ** |

Pour chacun des exemples présentés ici, les animaux des groupes témoins et les animaux traités ont reçu respectivement par voie intrapéritonéale (i.p.) 100 µl du véhicule ou une dose unique de 20 mg / kg de produit. 3 jours après l'injection les animaux sont euthanasiés, leur cerveau prélevé, congelé et échantillonné en coupes frontales de 20 µm. Dans la région contenant le LC un échantillonnage par intervalles de 80 µm est réalisé et les coupes sont transférées directement sur des filtres de nitrocellulose (Réf. 3). La TH est déterminée par immunochimie. Les résultats représentent la moyenne ± sem obtenue sur la totalité de la structure dans chacun des groupes traités. Ils sont exprimés en pourcentage de la valeur moyenne trouvée dans le groupe témoin correspondant. **p<0,02 ; *** p<0,002 ; **** p<0,0002.

Parmi les exemples cités des résultats complémentaires ont été obtenus avec le composé de formule (Ie1) pour laquelle l'administration aigue ou séquentielle fait apparaître une augmentation significative de la population de cellules noradrénergiques du locus caeruleus comme l'illustre la figure ci dessous :

Les résultats de la figure 2 représentent la distribution postéro-antérieure du nombre de cellules contenant la TH dans le LC déterminée par immunohistochimie obtenue dans un groupe témoin de souris Balb/c et dans un groupe de souris traitées par la molécule de formule (Ie1). Chaque barre représente la moyenne obtenue dans chaque intervalle anatomique de 80 µm dans chaque groupe expérimental ± sem. **** indique p<0,0001 déterminée par une ANOVA II (facteur traitement). On note l'augmentation très significative du nombre de cellules exprimant la TH. Dans la totalité de la structure les valeurs moyennes trouvées sont respectivement de 996±1 et 1250 ± 58 dans les groupes témoin et traité. (dose administrée : 20 mg/kg i.p. traitement séquentiel à J0, J3, J6, J9 et J.12 sacrifice des animaux à J16)

Ce phénomène peut être stabilisé par un traitement séquentiel approprié (par exemple : 1 injection i.p. 20mg/kg i.p. chaque 3 jours pendant 15 jours voir figure 2). Dans ces conditions, l'augmentation du nombre de cellules TH positives surnuméraires apparues dans le LC est encore maintenue 24 jours après l'arrêt du traitement.

Parallèlement à la réapparition des cellules dans le LC il est possible d'observer chez les animaux traités par le composé (Ie1), une augmentation très significative de la densité des fibres noradrénergiques dans le cortex préfrontal. (figure 3)

Les résultats de la figure 3 représentent la distribution des fibres TH positives sur un échantillon de cortex préfrontal de la souris Balb/c. A gauche : chez une souris témoin, on remarque au niveau de la couche moléculaire du cortex préfrontal, l'orientation typique (parallèle à la surface du cortex) des fibres noradrénergiques identifiées à l'aide d'une réaction immunocytochimique révélant la présence de la protéine TH. A droite : après un traitement séquentiel (5 injections i.p. chaque 3 jours de la molécules (Ie1); 20 mg/kg) la figure illustre l'augmentation de la densité des fibres TH positives identifiées dans la même région cérébrale. Les animaux ont été euthanasiés 3. jours après la dernière injection. Les lignes en pointillé marquent la limite de la couche moléculaire du cortex préfrontal.

Il a par ailleurs été montré :
- que la molécule (Ie1) est active par voie orale
- que cette activité est dose dépendante par voie intra-péritonéale (i.p.) et par voie orale (p.o.). Les doses efficaces 50% (DE50) sont respectivement de 0,5 et 1,5 mg/kg. La molécule de l'exemple préféré est ainsi 30 fois plus active que son précurseur de synthèse direct de formule (1) (voir Réf. 4, 5 et 6)

Au cours de la synthèse de la molécule (Ie) deux couples d'énantiomères sont séparés. Le couple d'énantiomères de formule (Ie1) a été séparé en deux produits purs (Ie1a et Ie1b). Le composé (Ie1b) est plus de deux fois plus actif que la forme (Ie1a) et est ainsi la forme préférée.

Les inventeurs ont mis également en évidence que les composés de formule (I), le radical R du composé (I) pouvant également représenter un atome d'hydrogène avec une double liaison entre les carbones 14 et 15, notamment le composé (Ie1) présentent une autre activité originale : l'activation de l'expression des hypocrétines dans une population spécifique de cellules de l'hypothalamus latéral qui n'expriment peu ou pas ces peptides chez la balb/c dans les conditions physiologiques normales. Cette propriété est partagée avec son précurseur de synthèse directe le composé de formule (1). Le tableau 3 ci-après illustre ce type de résultat pour les deux entités chimiques :

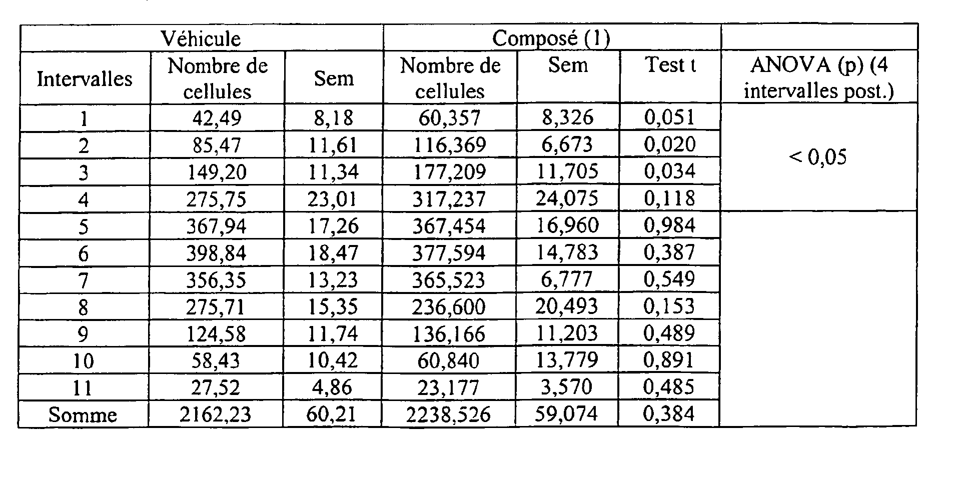

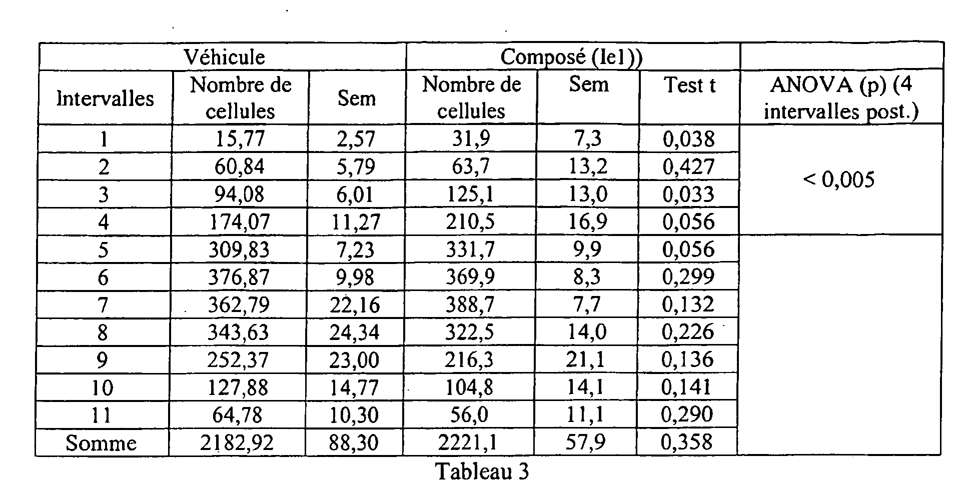

La distribution des cellules exprimant les hypocretines dans l'hypothalamus latéral est étudiée ici selon un échantillonnage postéro-antérieur par intervalles consécutifs de 80 µm d'épaisseur. Les cellules sont identifiées par immunocytochimie à l'aide d'anticorps spécifiques. Les deux molécules étudiées ont été administrées par voie i.p. à la dose unique de 20 mg/kg. Les animaux sont euthanasiés 3 jours après l'injection. Une population d'une centaine de cellules surnuméraires est ainsi identifiée au niveau du tiers postérieur du noyau cérébral.

Le test d'activité comportementale, le « Tail Suspension Test (TST) », validé par les antidépresseurs (Réf. 7) dans la souche Balb/c, a également été pratiqué pour la molécule de l'exemple préféré (Ie1b) à la dose de 1mg/kg i.p. L'expérience, donnée en exemple, a été réalisée 3 jours après une injection unique et l'effet comparé avec celui de l'imipramine (30 mg/kg i.p 30 minutes avant le test) (voir Tableau 4 ci-après)

**Tableau 4**

| | n | Dose administrée | Temps d'immobilisation (s) moyen | sem | P (test de student) |
|---|---|---|---|---|---|
| Véhicule | 8 | 0 | 180 | | |
| Composé (Ie1b) | 7 | 1mg/kg i.p. | 130 | 15,8 | 0,009 |
| Imipramine | 5 | 30mg/kg i.p. | 70 | 15,5 | 0,0001 |

Le temps d'immobilisation mesuré sur une période de 6 min. est significativement réduit 3 jours après une injection unique du composé (Ie1b), isomère actif du racémique (Ie1). Les résultats sont la moyenne de n animaux.

Un profil réceptoriel pratiqué « *in vitro* » sur 74 récepteurs et canaux à la concentration de 10 micromolaire du composé (Ie1) indique qu'il n'a pas d'interaction directe avec les canaux NA,K et Ca voltage dépendants et SK+Ca , ni avec les systèmes de transport des catécholamines, une liaison d'affinité modérée peut être suspectée pour les récepteur M1 et 5HT5A.

L'étude de la biodistribution de la molécule (Ie1) 45 minutes après administration i.p. et per os à des doses élevées (20 et 60 mg/kg respectivement) montre :
- qu'après administration i.p. de composé (Ie1) la présence de composé (1), (précurseur de synthèse) est indétectable.
- qu'après administration p.o., au même temps, le composé (Ie1) représente 89% de la concentration cérébrale et le composé 1 seulement 11%.
- que pour le composé (Ie1) le rapport concentration cérébrale / concentration hépatique est identique dans les deux voies d'administration (0,41 et 0,49 respectivement).

Ces résultats mettent en évidence que les composés selon la présente invention, le radical R du composé (1) pouvant également représenter un atome d'hydrogène avec une double liaison entre les carbones 14 et 15, et en particulier le composé préféré (Ie1), en particulier (Ie1b), sont spécifiquement responsables des effets observés décrits plus haut et que leur biodistribution cérébrale est favorable.

En conclusion les composés selon la présente invention, le radical R du composé (I) pouvant également représenter un atome d'hydrogène avec une double liaison entre les carbones 14 et 15, et en particulier le composé préféré (Ie1), en particulier (Ie1b), sont capables d'induire dans le cerveau un phénomène de plasticité qui permet la résurgence d'une population quiescente de cellules noradrénergiques du complexe céruléen dans le cerveau des souris de souche Balb/c. Ce réveil phénotypique est associé à une augmentation de l'innervation noradrénergique du cortex préfrontal dans cette lignée mutante génétiquement pure de souris qui présente une densité faible de cette innervation frontale. Un composé préféré a été identifié. Il est actif per os. Il est trente fois plus actif que son précurseur chimique direct qui présente des propriétés analogues. Ces composés selon la présente invention, le radical R du composé (I) pouvant également représenter un atome d'hydrogène avec une double liaison entre les carbones 14 et 15, sont également capables d'activer à long terme une population spécifique de neurones exprimant les hypocrétines dans l'hypothalamus latéral.

Compte tenu de l'efficacité potentielle de cette famille sur la médiation noradrénergique centrale, en particulier au niveau préfrontal, et sur les neurones hypocrétinergiques dont les implications dans le cycle veille sommeil et les modèles animaux de dépression sont connues (Réf. 8, 9). Compte tenu des contrôles particuliers qu'exercent, chez l'Homme, les neurones noradrénergiques dans le contrôle de l'humeur (Réf 10, 11) et des phénomènes cognitifs (Réf.12). Compte tenu des évidences directes de déficits fonctionnels frontaux et pré-frontaux dans plusieurs pathologies psychiatriques et neurologiques, des déficits déjà observés au niveau du locus caeruleus dans la dépression profonde (Réf.10, 11,12) et dans les grands syndromes dégénératifs du type Alzheimer et Parkinson (Réf.12, 13). Puisqu'il existe un nombre important de patients qui résistent aux thérapeutiques actuellement proposées, les composés selon la présente invention, le radical R du composé (I) pouvant également représenter un atome d'hydrogène avec une double liaison entre les carbones 14 et 15, et en particulier le composé préféré (Ie1), en particulier (Ie1b) sont utiles pour la prévention ou le traitement des syndromes dépressifs et en particulier des dépressions majeures, des dépressions résistantes telles que définies au DSM4 (Réf. 14, 15, 16) ainsi que pour la normalisation des symptômes négatifs des dépressions bipolaires (Réf.17) et des schizophrénies (Réf.18). Ce type de molécule est aussi utile pour le traitement des troubles symptomatiques frontaux dans leur composantes cognitives (Mild Cognitive Impairment (Ref. 19) ; états pré-démentiels et démentiels liés aux maladies d'Alzheimer ou de Parkinson (Réf. 20)) ou comportementales : hypoéveils, troubles de l'attention, troubles caractériels (type ADHD : »Attention Deficit-Hyperactivity Réf.21) ainsi que dans les troubles du sommeil en particulier dans les cas d'hypoéveils ou d'hypersomnies caractérisées (narcolepsie).

### Bibliographie

1. Ginovart N, Marcel D, Bezin L, Garcia C, Gagne C, Pujol JF, Weissmann D. Tyrosine hydroxylase expression within Balb/C and C57black/6 mouse locus coeruleus. I. Topological organization and phenotypic plasticity of the enzyme-containing cell population. Brain Res. 1996 May 20 ; 721(1-2):11-21
2. Ginovart N, Marcel D, Bezin L, Gagne C, Pujol JF, Weissmann D. Tyrosine hydroxylase expression within Balb/C and C57black/6 mouse locus coeruleus. II. Quantitative study of the enzyme level. Brain Res. 1996 May 6;719(1-2):45-55
3. Weissmann D, Labatut R, Richard F, Rousset C, Pujol JF. Direct transfer into nitrocellulose and quantitative radioautographic anatomical détermination of brain tyrosine hydroxylase protein concentration. J Neurochem. 1989 Sep;53 (3): 793-9.
4. Bourde O., Schmitt P and Pujol JF. Long term effect of RU24722 on tyrosine hydroxylase protein concentration in the locus coeruleus of mice: Differential results in Balb/C, C57BL/6 and their CB6 F1 hybrid. Neurochem Int. 1991, 19, (1), 25-31
5. Labatut R, Richard F, Milne B, Quintin L, Lecestre D, Pujol JF. Long-term effects of RU24722 on tyrosine hydroxylase of the rat brain. J Neurochem. 1988 Nov ; 51(5):1367-74.
6. Schmitt P, Reny-Palasse V, Bourde O, Garcia C, Pujol JF. Further characterization of the long-term effect of RU24722 on tyrosine hydroxylase in the rat locus coeruleus. J Neurochem. 1993 Oct;61(4):1423-9.
7. X. Liu and H.K. Gershenfeld : Genetic Differences in the Tail-Suspension Test and Its Relationship to Imipramine Response among 11 Inbred Strains of Mice : Biol. Psychiatry 2001 49, 575-581
8. J.S. Allard Y. Tizabio, JP Shaffery, C.O. Trouth, K. Manabe. Stereological analysis of hypothalamic hypocretin/orexin neurons in animal models of depression Neuropeptides 2004, 38, 311-315
9. D.Chabas,S.Taheri, C Renier, and E mignot. The genetic narcolepsie : An Rev. Genomics Human Genet. 2003, 4, 459-83
10. Arango V, Underwood MD, Mann J, Fewer pigmented locus coeruleus neurons in suicide victims : preliminary results. Biol Psychiatry 1996, 39 112-120
11. Mann J.J. Neurobiology of suicidal behavior , Nature Reviews/Neuroscience 4, 2003, 820-28
12. Marc R. Mariena,*, Francis C. Colpaerta, Alan C. Rosenquist Noradrenergic mechanisms in neurodegenerative diseases: a theory : Brain Research Reviews 45 (2004) 38 - 78
13. M. Gesi, P. Soldani, F. S. Giorgi, A. Santinami, 1. Bonaccorsi and F. Fornai The role of the locus coeruleus in the development of Parkinson's disease Neuroscience & Biobehavioral Reviews, Volume 24, Issue 6, August 2000, Pages 655-668
14. The fourth edition of the Diagnostic and Statistical Manual of mental Disorder.American Psychiatric Association Publisher.Washington DC
15. Fava M, Davidson KG.Definition and epidemiology of treatment-resistant depression Psychiatry Clin North Am. 1996 Jun;19(2): 179-20
16. Fava M, Davidson KG.Definition and epidemiology of treatment-resistant depression Psychiatry Clin North Am. 1996 Jun ; 19(2): 179-200
17. Kettera T A and Drevets W C , Clinical Neuroscience Research 2 (2002) 182-192 Neuroimaging studies of bipolar depression: functional neuropathology, treatment effects, and predictors of clinical response Clinical Neuroscience Research 2 (2002) 182-192
18. J. D. CohenR. GanguliC. CarterJ. BrarT. NicholsM. DeLeoM. Mintun: Hypofrontality and working memory dysfunction in schizophrenia Biological Psychiatry, Volume 37, Issue 9, 1 May 1995, Page 633
19. Ronald C. Petersen : Mild cognitive impairment : clinical trials : Nature reviews |Drug Discovery volume 2| august 2003 647
20. Vjera A. Holthoff, Bettina Beuthien-Baumann, Elke Kalbe, Susanne Lüdecke, Olaf Lenz, Gerhard Zündorf, Sebastian Spirling, Kristin Schierz, Peter Winiecki, Sandro Sorbi, and Karl Herholz : Regional Cerebral Metabolismin Early Alzheimer's Disease with Clinically Significant Apathy or Dépression : BIOL PSYCHIATRY 2005;57: 412-421
21. Eve M. Valera, Stephen V. Faraone, Joseph Biederman, Russell A. Poldrack, and Larry J. Seidman : Functional Neuroanatomy of Working Memory in Adults with Attention-Deficit/Hyperactivity Disorder : Biol Psychiatry 2005;57: 439-447

## Revendications

1. Composé répondant à la formule (1) 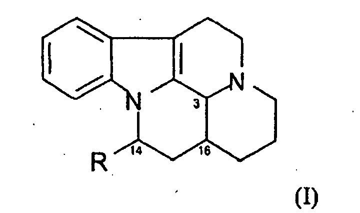 dans laquelle R représente un radical -AR', dans lequel A représente un hétéroatome choisi parmi l'azote et l'oxygène, et R' représente
■ un groupement choisi dans le groupe constitué par les radicaux alkyles en C₁-C₆, alcényles en C₁-C₆, alcynyles en C₁-C₆, arylalkyles, alcoxyarylalkyles, hétéroarylakyles et hétérocycloalkyles, linéaires ou ramifiés ;
■ les esters de formule -R₁-CO-O-R₂, dans laquelle R₁ représente un radical choisi dans le groupe constitué par les radicaux alkylènes en C₁-C₆, alcénylènes en C₂-C₆ ou alcynylènes en C₂-C₆, linéaires ou ramifiés, et R₂ représente un radical choisi dans le groupe constitué par l'hydrogène, les radicaux alkyles en C₁-C₆, alcényles en C₂-C₆, alcynyles en C₂-C₆, linéaires ou ramifiés, cycloalkyles en C₃-C₁₂ ;
■ les amides de formule 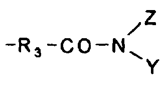 dans laquelle R₃ représente un radical choisi dans le groupe constitué par les radicaux alkyles en C₁-C₆, alcénylènes en C₂-C₆ ou alcynylènes en C₂-C₆, linéaires ou ramifiés, et Y représente un radical choisi dans le groupe constitué par l'hydrogène, les radicaux alkyles en C₁-C₆, alcényles en C₂-C₆, alcynyles en C₂-C₆, aryles, arylaklyles, hétéroarylalkyles ou hétérocycloalkyles, linéaires ou ramifiés, et Z représente un radical choisi dans le groupe constitué par l'hydrogène, les radicaux alkyles en C₁-C₆, alcynyles en C₂-C₆, alcynyles en C₂-C₆, aryles, arylaklyles, hétéroarylalkyles ou hétérocycloalkyles, linéaires ou ramifiés, Y et.Z pouvant former ensemble un radical cycloalkyle en C₃-C₆ ou un radical hétérocyclique en C₃-C₆, éventuellement substitué par un ou plusieurs radicaux alkyles en C₁-C₆ aryles, hétéroaryles ou halogènes ;
■ un radical choisi dans le groupe constitué par les radicaux alkyles en C₂-C₆, alcényles en C₂-C₆ ou alcynyles en C₁-C₆, linéaires ou ramifiées, substitués par au moins une amine de formule 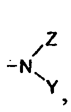 dans laquelle Y et Z sont tels que définis précédemment ;
ou l'un de ses sels pharmaceutiquement acceptables, y compris ses isomères, ses énantiomères, ses diastéréoisomères et leurs mélanges.

2. Composé selon la revendication 1, **caractérisé en ce que** R' représente un groupement de formule R₁-CO-O-R₂, dans laquelle R₁ représente un radical alkylène en C₁-C₆, avantageusement -CH₂-.

3. Composé selon l'une quelconque des revendications précédentes, **caractérisé en ce que** R₂ représente un atome d'hydrogène ou un radical alkyle en C₁-C₆.

4. Composé selon l'une quelconque des revendications précédentes, **caractérisé en ce que** -R' représente un amide de formule 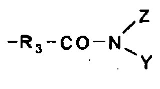 dans laquelle Y et Z forment ensemble un radical hétérocyclique en C₃-C₆, éventuellement substitué par un ou plusieurs radicaux alkyles en C₁-C₆.

5. Composé selon l'une quelconque des revendications précédentes, **caractérisé en ce que** -R' représente un radical aminoalkyle choisi dans le groupe constitué par les radicaux alkyles en C₁-C₆ substitués par au moins une amine de formule 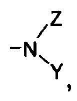 dans laquelle , Y et Z forment ensemble un radical hétérocyclique en C₂-C₆, éventuellement substitué par un ou plusieurs radicaux alkyles en C₁-C₆.

6. Composé selon l'une quelconque des revendications précédentes, **caractérisé en ce que** -R' représente un radical hétéroarylalkyle.

7. Composé selon l'une quelconque des revendications précédentes, **caractérisé en ce qu'**il répond à une des formules suivantes :

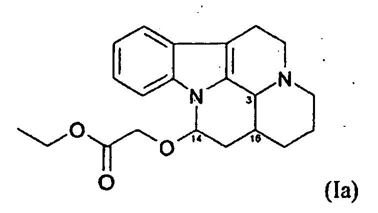

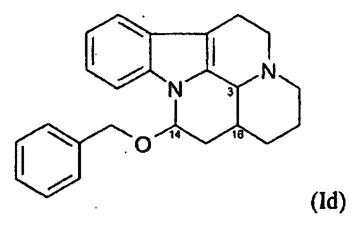

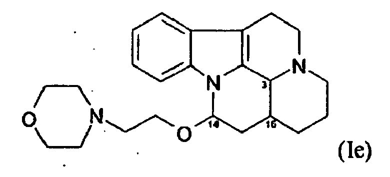

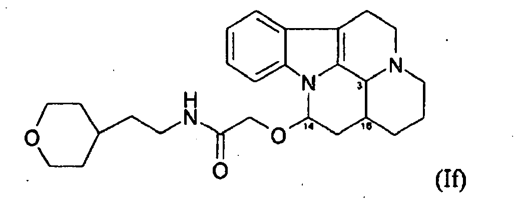

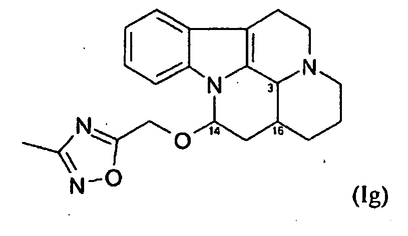

8. Composé selon l'une quelconque des revendications précédentes, **caractérisé en ce que** l'atome d'hydrogène en position 3 et l'atome d'hydrogène en position 16 sont *trans,* le cas échéant le radical R en position 14 pouvant être quant à lui sous la forme α ou β.

9. Composé selon l'une quelconque des revendications précédentes, **caractérisé en ce que** le composé de formule (I) ou l'un de ses sels pharmaceutiquement acceptables est sous la forme d'un mélange racémique ou optiquement actif

10. Composé selon l'une quelconque des revendications précédentes, **caractérisé en ce que** ledit composé de formule (I) ou l'un de ses sels pharmaceutiquement acceptables est choisi parmi les composés de formule (I) suivants :
a) les composés sous la forme (3α) dextrogyres et/ou lévogyres ; et
b) les composés sous la forme (16α) dextrogyres et/ou lévogyres,
et dans lequel, le mélange des deux diastéréoisomères lévogyres et dextrogyres présents dans ces composés a) et b) est en proportion équimolaire ou non.

11. Composé selon l'une quelconque des revendications précédentes, **caractérisé en ce que** ledit composé de formule (I) ou l'un de ses sels pharmaceutiquement acceptables est choisi parmi les composés de formule (I) :
a) les composés sous la forme (3α, 14α);
b) les composés sous la forme (3α, 14β);
c) les composés sous la forme (14α, 16α); et
d) les composés sous la forme (14β, 16α).

12. Composé selon l'une quelconque des revendications 7 à 10, **caractérisé en ce que** ledit composé de formule (I) ou l'un de ses sels pharmaceutiquement acceptables représente l'épimère (Ie1) comprenant le couple d'énantiomères (3α, 14β) et (16α, 14α) de formules suivantes :

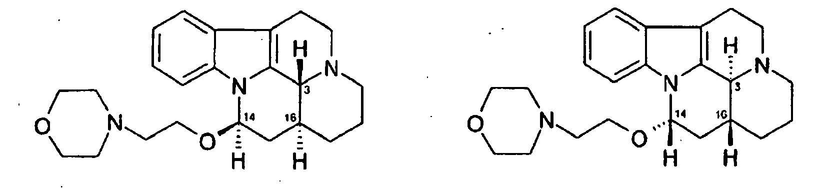

13. Composé selon la revendication 12, **caractérisé en ce que** ledit composé de formule (1) ou l'un de ses sels pharmaceutiquement acceptables représente l'énantiomère (Ie1b) qui est élué en second lorsque le mélange d'énantiomères (Ie1) est sownis à une chromatographie HPLC sur une colonne dont la phase stationnaire est constituée de particules de gel de silice sur lesquelles est greffée de la cellulose tris(2,5-diméthylphénylcarbamate), la phase mobile utilisée étant de l'acétonitrile.

14. Composé selon l'une des revendications 1 à 13 pour son utilisation comme médicament.

15. Composition pharmaceutique comprenant un composé selon l'une quelconque des revendications 1 à 14 et un excipient pharmaceutiquement acceptable.

16. Utilisation d'un composé selon la revendication 14 ou d'une composition selon la revendication 15, pour la préparation d'une composition pharmaceutique destinée au traitement et/ou la prévention de la dépression.

17. Utilisation d'un composé selon la revendication 14 ou d'une composition selon la revendication 15, pour la préparation d'une composition pharmaceutique destinée au traitement et/ou la prévention des dépressions majeures.

18. Utilisation d'un composé selon la revendication 13 ou 14, ou d'une composition selon la revendication 15, pour la préparation d'une composition pharmaceutique destinée au traitement et/ou la prévention de la dépression bipolaire et/ou de la schizophrénie.

19. Utilisation d'un composé selon la revendication 13 ou 14, ou d'une composition selon la revendication 15, pour la préparation d'une composition pharmaceutique destinée au traitement et/ou la prévention des désordres du cycle veille-sommeil ou troubles du sommeil.

20. Utilisation selon la revendication 19, **caractérisée en que** les désordres du cycle veille-sommeil ou troubles du sommeil sont choisis parmi l'hypoéveil ou l'hypersomnie caractérisée (narcolepsie).

21. Utilisation d'un composé selon la revendication 13 ou 14, ou d'une composition selon la revendication 15 pour la préparation d'une composition pharmaceutique destinée au traitement et/ou la prévention pour le traitement des troubles symptomatiques frontaux dans leurs composantes cognitives.

22. Utilisation selon la revendication 21, **caractérisée en ce que** les troubles symptomatiques frontaux dans leurs composantes cognitives sont choisis parmi les états pré-démentiels et démentiels liés aux maladies d'Alzheimer ou de Parkinson, aux maladies comportementales.

23. Utilisation selon la revendication 22, **caractérisée en ce que** les maladies comportementales sont choisies parmi les troubles de l'attention, troubles caractériels (type ADHD : »Attention Deficit-Hyperactivity Ref.21).

24. Utilisation selon la revendication 21, pour la préparation d'une composition pharmaceutique destinée au traitement et/ou la prévention des troubles de la mémoire, notamment liés au vieillissement ou liés aux maladies d'Alzheimer ou de Parkinson.

25. Utilisation selon l'une des revendications 16 à 24, **caractérisée en ce que** ledit composé est administré par voie orale, par voie intraveineuse, par voie intrapéritonéale ou par voie intramusculaire.

26. Utilisation selon l'une des revendications 16 à 25, **caractérisée en ce que** ledit composé est administré à des doses de 20 à 60 mg par jour chez le patient à traiter.

## Claims

1. Compound satisfying formula (I) 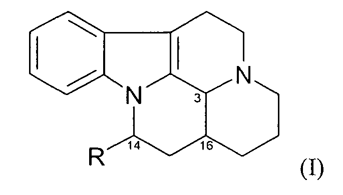 in which R represents a -AR' radical, in which A represents a heteroatom and R' represents
■ a group chosen from the group composed of alkyl radicals in C₁-C₆, alkenyl radicals in C₁-C₆, alkynyl radicals in C₁-C₆, arylalkyl radicals, alcooxyarylalkyls, heteroarylakyls and heterocycloalkyls, linear or branched;
■ esters with formula -R₁-CO-O-R₂, in which R₁ represents a radical chosen from the group composed of alkylene radicals in C₁-C₆, alkenylene radicals in C₂-C₆ or alkynylene radicals in C₂-C₆, linear or branched, and R₂ represents a radical chosen among the group composed of hydrogen, alkyl radicals in C₁-C₆, alkenyl radicals in C₂-C₆, alkynyl radicals in C₂-C₆, linear or branched or cycloalkyl radicals in C₃-C₁₂;
■ amides with formula 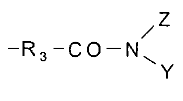 in which R₃ represents a radical chosen from the group composed of alkyl radicals in C₁-C₆, alkenylene radicals in C₂-C₆ or alkynylene radicals in C₂-C₆, linear or branched, and Y represents a radical chosen from the group composed of hydrogen, alkyl radicals in C₁-C₆, alkenyl radicals in C₂-C₆, alkynyl radicals in C₂-C₆, aryls, arylalkyls, heteroarylalkyls or heterocycloalkyls, linear or branched, and Z represents a radical chosen from the group composed of hydrogen, alkyl radicals in C₁-C₆, alkenyl radicals in C₂-C₆, alkynyl radicals in C₂-C₆, aryls, arylalkyles, heteroarylalkyls or heterocycloalkyls, linear or branched, Y and Z together possibly forming a cycloalkyl radical in C₃-C₆ or an heterocyclic radical in C₃-C₆, possibly substituted by one or several alkyl radicals in C₁-C₆, aryls, heteroaryls or halogen radicals;
■ a radical chosen from the group composed of alkyl radicals in C₂-C₆, alkenyl radicals in C₂-C₆ or alkynyl radicals in C₁-C₆, linear or branched, substituted by at least one amine with formula 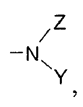 in which Y and Z are as defined above; or one of its pharmaceutically acceptable salts, including its isomers, enantiomers, diastereoisomers and mixes of them.

2. Compound according to claim 1, **characterised in that** R' represents a group with formula R₁-CO-O-R₂, in which R₁ represents an alkylene radical in C₁-C₆, advantageously the -CH₂- radical.

3. Compound according to any one of the previous claims, **characterised in that** R₂ represents a hydrogen atom, or an alkyl radical in C₁-C₆.

4. Compound according to any one of the previous claims, **characterised in that** R' represents an amide with formula 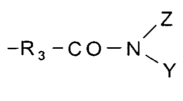 in which Y and Z together form a heterocyclic radical in C₃-C₆, possibly substituted by one or several alkyl radicals in C₁-C₆.

5. Compound according to any one of the previous claims, **characterised in that** -R' represents an amino-alkyl radical selected from the group composed of alkyl radicals in C₁-C₆ substituted by at least one amine with formula 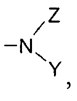 in which Y and Z together form a heterocyclic radical in C₂-C₆ possibly substituted by one or several alkyl radicals in C₁-C₆.

6. Compound according to any one of the previous claims, **characterised in that** -R' represents a heteroarylalkyl radical.

7. Compound according to any one of the previous claims, **characterised in that** it satisfies the following formulas:

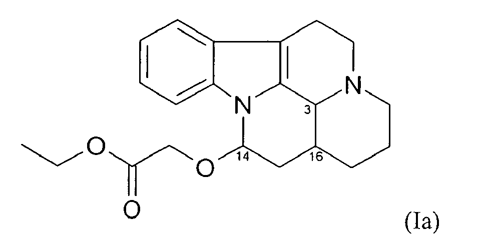

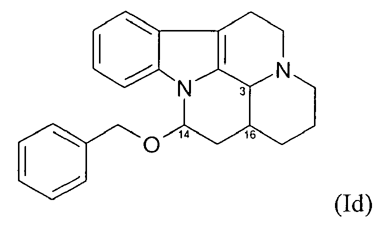

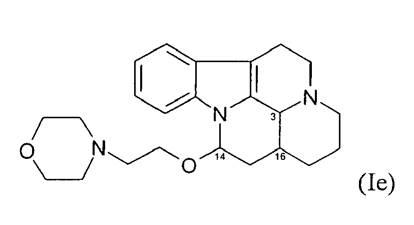

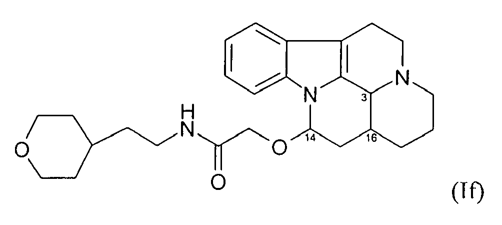

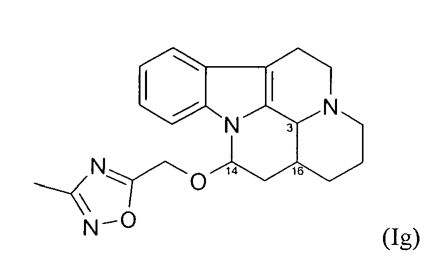

8. Compound according to any one of the previous claims, **characterised in that** the hydrogen atom in position 3 and the hydrogen atom in position 16 are in the *trans* position, and the radical R in position 14 may be in α or β form.

9. Compound according to any one of the previous claims, **characterised in that** the compound with formula (I) or one of its pharmaceutically acceptable salts is in the form of a racemic or optically active mix.

10. Compound according to any one of the previous claims, **characterised in that** the said compound with formula (I) or one of its pharmaceutically acceptable salts is chosen from the following compounds with formula (I):
a) compounds in the dextrogyre and/or levogyre form (3α); and
b) compounds in the dextrogyre and/or levogyre form (16α),
and in which the mix of the two levogyre and dextrogyre diastereoisomers present in these compounds a) and b) is or is not in an equimolar proportion.

11. Compound according to any one of the previous claims, **characterised in that** the said compound with formula (I) or one of its pharmaceutically acceptable salts is chosen from the compounds with formula (I):
a) compounds in the form (3α, 14α);
b) compounds in the form (3α, 14β);
c) compounds in the form (14α, 16α); and
d) compounds in the form (14β, 16α).

12. Compound according to any one of claims 7 to 10, **characterised in that** the said compound with formula (I) or one of its pharmaceutically acceptable salts represents the epimer (Ie1) comprising the enantiomer pair (3α, 14β) and (16α, 14α) with the following formulas:

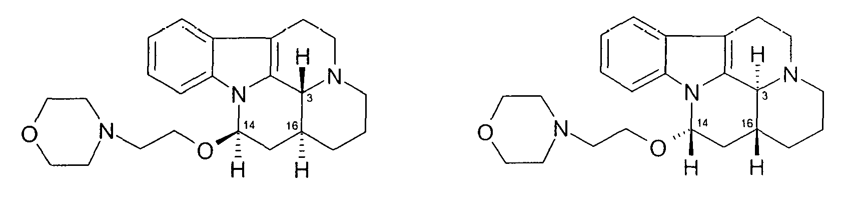

13. Compound according to claim 12, **characterised in that** the said compound with formula (I) or one of its pharmaceutically acceptable salts represents the enantiomer (Ie1b) that is the second eluted compound when the enantiomer mix (Ie1) is subjected to an HPLC chromatography using a column in which the stationary phase is composed of silica gel particles on which tris(2,5-dimethylphenylcarbamate) cellulose is grafted, the mobile phase used being acetonitrile.

14. Compound according to claims 1 to 13, for its use as a drug.

15. Pharmaceutical composition comprising a compound according to any one of claims 1 to 14 and a pharmaceutically acceptable excipient.

16. Use of a compound according to claim 14, or a composition according to claim 15, for the preparation of a pharmaceutical composition intended for the treatment and/or prevention of depression.

17. Use of a compound according to claim 14 or a composition according to claim 15 for the preparation of a pharmaceutical composition intended for the treatment and/or prevention of major depressive disorders.

18. Use of a compound according to claim 13 or 14 or a composition according to claim 15 for the preparation of a pharmaceutical composition intended for the treatment and/or prevention of bipolar depression or schizophrenia.

19. Use of a compound according to claim 13 or 14 or a composition according to claim 15 for the preparation of a pharmaceutical composition intended for the treatment and/or prevention of wakening-sleep cycle disorders or sleep disorders.

20. Use according to claim 19, **characterised in that** the wakening-sleep cycle disorders or sleep disorders are chosen among the group composed of hypo arousal or characterised hypersomnia (narcolepsy).

21. Use of a compound according to claim 13 or 14 or a composition according to claim 15 for the preparation of a pharmaceutical composition intended for treatment and/or prevention for the treatment of cognitive aspects of symptomatic frontal disorders.

22. Use according to claim 21, **characterised in that** the cognitive components of symptomatic frontal disorders are chosen from among pre-demential and demential conditions related to Alzheimer's disease or Parkinson's disease, or behavioural diseases.

23. Use according to claim 22, **characterised in that** the behavioural diseases are chosen from among attention problems, character disorders (ADHD type: "Attention Deficit - Hyperactivity" Ref. 21).

24. Use according to claim 21, for the preparation of a pharmaceutical composition intended for the treatment and/or prevention of memory disorders, particularly related to ageing or to Alzheimer's disease and Parkinson's disease.

25. Use according to one of claims 16 to 24, **characterised in that** the said compound is administered by oral, intravenous, intraperitoneal or intramuscular route.

26. Use according to one of claims 16 to 25, **characterised in that** the said compound is administered to the patient to be treated every day in doses of 20 to 60 mg.

## Patentansprüche

1. Verbindung, entsprechend der folgenden Formel (I) : 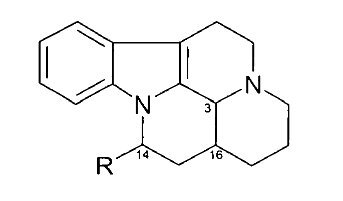 wobei R ein -AR'-Radikal darstellt, wobei A ein Heteroatom darstellt, das unter Stickstoff und Sauerstoff ausgewählt wird, und R' Folgendes darstellt:
- eine Gruppierung, ausgewählt aus der Gruppe bestehend aus den C₁-C₆-Alkyl-, C₁-C₆-Alkenyl-, C₁-C₆-Alkynyl-, Arylalkyl-, Alkoxyarylalkyl-, Heteroarylalkyl- und Heterozykloalkylradikalen, unverzeigt oder verzweigt;
- die Ester der Formel -R₁-CO-O-R₂, wobei R₁ ein Radikal darstellt, ausgewählt aus der Gruppe bestehend aus den C₁-C₆-Alkylen-, C₂-C₆-Alkenylen- oder C₂-C₆-Alkynylenradikalen, unverzweigt oder verzweigt, und R₂ ein Radikal darstellt, ausgewählt aus der Gruppe bestehend aus Wasserstoff, den C₁-C₆-Alkyl-, C₂-C₆-Alkenyl-, C₂-C₆-Alkynylradikalen, unverzweigt oder verzweigt, C₃-C₁₂_Zykloalkylradikal;
- die Amide der Formel 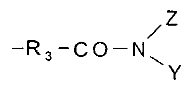 wobei R₃ ein Radikal darstellt, ausgewählt aus der Gruppe bestehend aus den C₁-C₆-Alkyl- C₂-C₆-Alkenylen- oder C₂-C₆-Alkynylenradikalen, unverzweigt oder verzweigt, und Y ein Radikal darstellt, ausgewählt aus der Gruppe bestehend aus Wasserstoff, den C₁-C₆-Alkyl- , C₂-C₆-Alkenyl-, C₂-C₆-Alkynyl-, Aryl-, Arylaklyl-, Heteroarylalkyl- oder Heterozykloalkylradikalen, unverzweigt oder verzweigt, und Z ein Radikal darstellt, ausgewählt aus der Gruppe bestehend aus Wasserstoff, den C₁-C₆-Alkyl-, C₂-C₆-Alkenyl-, C₂-C₆-Alkynyl-, Aryl-, Arylalkyl-, Heteroarylalkyl- oder Heterozykloalkylradikalen, unverzweigt oder verzweigt, wobei Y und Z zusammen ein C₃-C₆-Zykloalkylradikal oder ein heterozyklisches C₃-C₆-Radikal bilden können, eventuell ersetzt durch ein oder mehrere C₁-C₆-Alkyl-, Aryl-, Heteroaryl- oder Halogenradikale ;
- ein Radikal, ausgewählt aus der Gruppe bestehend aus den C₂-C₆-Alkyl-, C₂-C₆-Alkenyl- oder C₁-C₆-Alkynylradikalen, unverzweigt oder verzweigt, ersetzt durch
zumindest ein Amin der Formel 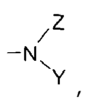 wobei Y und Z wie oben definiert sind;
oder eines ihrer pharmazeutisch annehmbaren Salze, darin eingeschlossen ihre Isomere, ihre Enantiomere, ihre Diastereoisomere und ihre Mischungen.

2. Verbindung nach Anspruch 1, **dadurch gekennzeichnet, dass** R' eine Gruppierung mit der Formel R₁-CO-O-R₂ darstellt, wobei R₁ ein C₁-C₆-Alkylenradikal darstellt, vorzugsweise -CH₂-.

3. Verbindung nach einem der vorhergehenden Ansprüche, **dadurch gekennzeichnet, dass** R₂ ein Wasserstoffatom oder ein C₁-C₆-Alkylradikal darstellt

4. Verbindung nach einem der vorhergehenden Ansprüche, **dadurch gekennzeichnet, dass** -R' ein Amid
der Formel 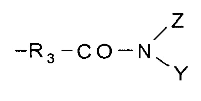 darstellt, wobei Y und Z zusammen ein heterozyklisches C₃-C₆-Radikal darstellen, eventuell ersetzt durch ein oder mehrere C₁-C₆-Alkylradikale.

5. Verbindung nach einem der vorhergehenden Ansprüche, **dadurch gekennzeichnet, dass** -R' ein Aminoalkylradikal darstellt, ausgewählt aus der Gruppe bestehend aus den C₁-C₆ Alkylradikalen, ersetzt durch
zumindest ein Amin der Formel 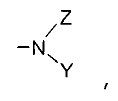 wobei Y und Z zusammen ein heterozyklisches C₂-C₆ Radikal bilden, eventuell ersetzt durch ein oder mehrere C₁-C₆-Alkylradikale.

6. Verbindung nach einem der vorhergehenden Ansprüche, **dadurch gekennzeichnet, dass** -R' ein Heteroarylalkylradikal darstellt.

7. Verbindung nach einem der vorhergehenden Ansprüche, **dadurch gekennzeichnet, dass** sie einer der folgenden Formeln entspricht:

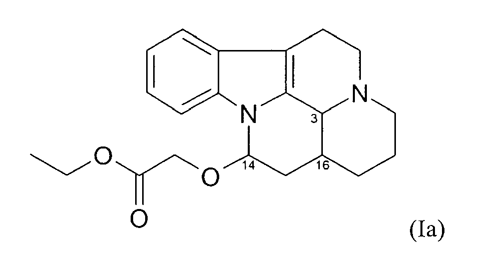

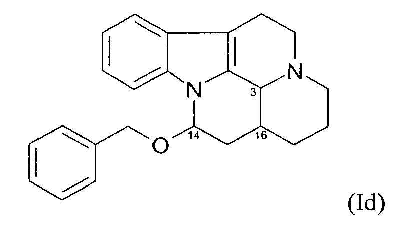

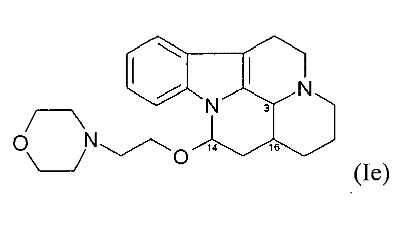

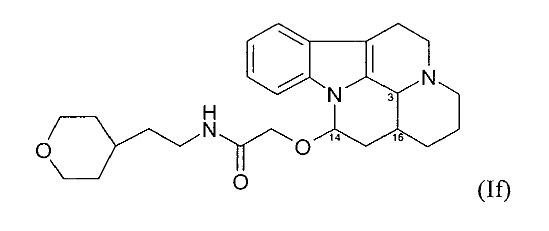

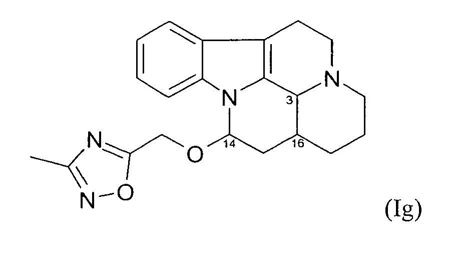

8. Verbindung nach einem der vorhergehenden Ansprüche, **dadurch gekennzeichnet, dass** das Wasserstoffatom in Position 3 und das Wasserstoffatom in Position 16 *trans* sind, wobei gegebenenfalls das Radikal R in Position 14 seinerseits die Form α oder β aufweisen kann.

9. Verbindung nach einem der vorhergehenden Ansprüche, **dadurch gekennzeichnet, dass** die Verbindung der Formel (I) oder eines ihrer pharmazeutisch annehmbaren Salze die Form einer racemischen oder optisch aktiven Mischung aufweist.

10. Verbindung nach einem der vorhergehenden Ansprüche, **dadurch gekennzeichnet, dass** die Verbindung der Formel (I) oder eines ihrer pharmazeutisch annehmbaren Salze unter den folgenden Verbindungen der Formel (I) ausgewählt wird:
a) den Verbindungen mit der rechtsdrehenden und/oder linksdrehenden Form (3α); und
b) den Verbindungen mit der rechtsdrehenden und/oder linksdrehenden Form (16α),
und wobei die Mischung der zwei linksdrehenden und rechtsdrehenden Diastereoisomere, die in diesen Verbindungen a) und b) vorhanden sind, in equimolärem Verhältnis vorliegt oder nicht.

11. Verbindung nach einem der vorhergehenden Ansprüche, **dadurch gekennzeichnet, dass** die Verbindung der Formel (I) oder eines ihrer pharmazeutisch annehmbaren Salze unter den folgenden Verbindungen der Formel (I) ausgewählt wird:
a) den Verbindungen mit der Form (3α, 14α);
b) den Verbindungen mit der Form (3α, 14β);
c) den Verbindungen mit der Form (14α, 16α); und
d) den Verbindungen mit der Form (14β, 16α).

12. Verbindung nach einem der Ansprüche 7 bis 10, **dadurch gekennzeichnet, dass** die Verbindung der Formel (I) oder eines ihrer pharmazeutisch annehmbaren Salze das Epimer (Ie1) darstellt, umfassend das Paar von Enantiomeren (3α, 14β) und (16α, 14α) der folgenden Formeln:

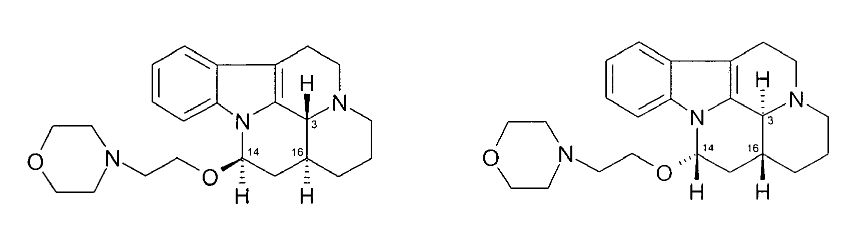

13. Verbindung nach Anspruch 12, **dadurch gekennzeichnet, dass** die Verbindung der Formel (I) oder eines seiner pharmazeutisch annehmbaren Salze das Enantiomer (Ie1b) darstellt, des als zweites ausgewählt wird, wenn die Mischung von Enantiomeren (Ie1) einer HPLC-Chromatographie auf einer Säule unterzogen wird, deren stationäre Phase aus Siliziumgelpartikeln besteht, auf denen Tris(2,5- dimethylphenylcarbamat)-Zellulose aufgepfropft ist, wobei die verwendete mobile Phase Acetonitril ist.

14. Verbindung nach einem der Ansprüche 1 bis 13 für ihre Verwendung als Arzneimittel.

15. Pharmazeutische Zusammensetzung, umfassend eine Verbindung nach einem der Ansprüche 1 bis 14 und einen pharmazeutisch annehmbaren Hilfsstoff.

16. Verwendung einer Verbindung nach Anspruch 14 oder einer Zusammensetzung nach Anspruch 15 zur
Zubereitung einer pharmazeutischen Zusammensetzung, die für die Behandlung und/oder die Vorbeugung der Depression bestimmt ist.

17. Verwendung einer Verbindung nach Anspruch 14 oder einer Zusammensetzung nach Anspruch 15 zur Zubereitung einer pharmazeutischen Zusammensetzung, die für die Behandlung und/oder die Vorbeugung von starken Depressionen bestimmt ist.

18. Verwendung einer Verbindung nach Anspruch 13 oder 14 oder einer Zusammensetzung nach Anspruch 15 zur Zubereitung einer pharmazeutischen Zusammensetzung, die für die Behandlung und/oder die Vorbeugung der bipolaren Depression und/oder der Schizophrenie bestimmt ist.

19. Verwendung einer Verbindung nach Anspruch 13 oder 14 oder einer Zusammensetzung nach Anspruch 15 zur Zubereitung einer pharmazeutischen Zusammensetzung, die für die Behandlung und/oder die Vorbeugung von Störungen des Wach-Schlaf-Zyklus oder Schlafstörungen bestimmt ist.

20. Verwendung nach Anspruch 19, **dadurch gekennzeichnet, dass** die Störungen des Wasch-Schlaf-Zyklus oder Schafstörungen unter dem Hyperwachzustand oder dem Hyperschlafzustand (Narcolepesie) ausgewählt werden.

21. Verwendung einer Verbindung nach Anspruch 13 oder 14 oder einer Zusammensetzung nach Anspruch 15 zur Zubereitung einer pharmazeutischen Zusammensetzung, die für die Behandlung und/oder die Vorbeugung für die Behandlung der symptomatischen frontalen Störungen in ihren kognitiven Bestandteilen bestimmt ist.

22. Verwendung nach Anspruch 21, **dadurch gekennzeichnet, dass** die symptomatischen frontalen Störungen in ihren kognitiven Bestandteilen unter den vordemenziellen und demenziellen Zuständen, die mit der Alzheimer'schen oder der Parkinson'schen Krankheit, mit Verhaltensstörungen verbunden sind, ausgewählt werden.

23. Verwendung nach Anspruch 22, **dadurch gekennzeichnet, dass** die Verhaltensstörungen unter den Aufmerksamkeitsstörungen, Charakterstörungen (Typ ADHD: "Attention Deficit-Hyperactivity Ref.21) ausgewählt werden.

24. Verwendung nach Anspruch 21 für die Zubereitung einer pharmazeutischen Zusammensetzung, die für die Behandlung und/oder die Vorbeugung der Gedächtnisstörungen, insbesondere verbunden mit der Alterung oder verbunden mit der Alzheimer'schen oder der Parkinson'schen Krankheit bestimmt ist.

25. Verwendung nach einem der Ansprüche 16 bis 24, **dadurch gekennzeichnet, dass** die Verbindung auf oralem Weg, intravenösem Weg, intraperitonealem Weg oder intramuskulärem Weg verabreicht wird.

26. Verwendung nach einem der Ansprüche 16 bis 25, **dadurch gekennzeichnet, dass** die Verbindung in Dosen zwischen 20 und 60 mg pro Tag bei den zu behandelnden Patienten verabreicht wird.
